# EUROPEAN PATENT SPECIFICATION

(11) **EP 3 329 962 B1**
(45) Date of publication and mention of the grant of the patent: **13.05.2020**
(21) Application number: 17201924.2
(22) Date of filing: 15.11.2017
(51) Int. Cl.: A61N 1/372, A61B 34/20, A61B 17/24, A61B 5/06, A61N 1/36, A61N 1/05, A61B 17/34, A61B 17/00, A61B 90/00, A61B 34/10

(54) **IMPLANT AND DELIVERY SYSTEM FOR NEURAL STIMULATOR**
IMPLANTAT UND IMPLANTATIONSSYSTEM FÜR NERVENSTIMULATOR
IMPLANT ET SYSTÈME D'IMPLANTATION POUR STIMULATEUR NEURAL

(30) Priority: 15.11.2016 US 201662422244 P
(43) Date of publication of application: 06.06.2018
(73) Proprietor: Brainsgate Ltd., Caesarea (IL)
(72) Inventor: DAYAN, Avinoam, Zichron Yaakov (IL); DVORSKY, Israel, Kfar Saba (IL); SHAI, Eyal, Pardes Hana (IL)
(74) Representative: White, Duncan Rohan

(56) References cited:
- EP-A1- 2 186 474
- EP-A2- 1 743 575
- EP-A2- 2 878 335
- WO-A1-2012/151412
- WO-A1-2014/037524
- US-A1- 2006 085 041
- US-A1- 2010 114 184

## Description

### FIELD OF THE APPLICATION

Some applications of the invention relate generally to medical procedures and implantable devices. More specifically, some applications of the invention relate to the use of electrical devices for implantation in the head.

### BACKGROUND

Surgical guides are typically generated based on computed tomography (CT) image data, and provide a dentist with guidance as to an optimal location for drilling into a jaw bone of a subject during implantation of dental implants.

US-B- 7,120,489, which is assigned to the assignee of the present patent application, describes apparatus for modifying a property of a brain of a patient, including electrodes applied to a sphenopalatine ganglion (SPG) or a neural tract originating in or leading to the SPG. A control unit drives the electrodes to apply a current capable of inducing (a) an increase in permeability of a blood-brain barrier (BBB) of the patient, (b) a change in cerebral blood flow of the patient, and/or (c) an inhibition of parasympathetic activity of the SPG.

US-B-7,117,033 describes a method for treating a subject, comprising positioning at least one electrode at least one site of the subject for less than about 3 hours, applying an electrical current to the site of the subject, and configuring the current to increase cerebral blood flow (CBF) of the subject, so as to treat a condition of the subject. The site is selected from the list consisting of: a sphenopalatine ganglion (SPG) of the subject, a greater palatine nerve of the subject, a lesser palatine nerve of the subject, a sphenopalatine nerve of the subject, a communicating branch between a maxillary nerve and an SPG of the subject, an otic ganglion of the subject, an afferent fiber going into the otic ganglion of the subject, an efferent fiber going out of the otic ganglion of the subject, an infraorbital nerve of the subject, a vidian nerve of the subject, a greater superficial petrosal nerve of the subject, and a lesser deep petrosal nerve of the subject.

US-B-7,561,919 describes apparatus for application to a subject, including an elongated support element having a length of between 1.8 cm and 4 cm, and having proximal and distal ends; and one or more electrodes fixed to the support element in a vicinity of the distal end thereof, and adapted to apply an electrical current to a sphenopalatine ganglion (SPG) of the subject. The apparatus further includes a receiver, fixed to the support element, and electrically coupled to the electrodes; and a wireless transmitter, adapted to be placed in an oral cavity of the subject, and to be wirelessly coupled to the receiver. Other embodiments are also described.

US-A-2010/0114184 discloses apparatus including a surgical tool, which includes a proximal shaft, a distal rod, a proximal end thereof which is coupled to a distal end of the proximal shaft such that the distal rod articulates with the proximal shaft, and a target site, which comprises an aiming element, which is coupled to the distal rod and extends toward the proximal shaft, and which is indicative of an alignment of the distal rod with respect to the proximal shaft.

### SUMMARY OF APPLICATIONS

In some applications, a system is provided for delivery of a neural stimulator implant for electrical stimulation of a sphenopalatine ganglion (SPG) of a subject. Stimulation of the SPG typically treats various acute brain hypoperfusion states, such as occur during acute ischemic stroke. Typically, the system includes apparatus comprising an implantable neural stimulator, a steerable delivery guide, and an oral surgical guide.

The neural stimulator implant is configured to be passed through a greater palatine foramen of a palate of an oral cavity of a subject into a greater palatine canal, such that the neural stimulator implant is brought into a vicinity of a sphenopalatine ganglion (SPG), for example, into contact with the SPG. For some applications, the implant is a flexible implant configured to conform to the anatomical structure of the greater palatine canal, to facilitate advancement therethrough. For some applications, the implant comprises at least one electrode for stimulation of the SPG.

The neural stimulator implant is typically coupled to the steerable delivery guide. For some applications, a distal end of the steerable delivery guide is configured to puncture oral mucosa of the subject, allowing the neural stimulator implant to be passed through the palate in a minimally-invasive procedure, without requiring a prior surgical incision in the mucosa. Typically, the distal end of the steerable delivery guide is also configured to be passed through the greater palatine foramen into the greater palatine canal. The delivery guide is steered in the canal in order to deliver the neural stimulator implant to the SPG.

Typically, the surgical guide is generated based on CT data obtained by imaging the subject. Based on the CT data, the surgical guide is formed to provide a guide hole for locating the entrance to the greater palatine canal, such that the implantable neural stimulator may be passed through the guide hole and then into the greater palatine canal. In particular, the surgical guide is typically configured for placement on the subject's dental arch, such that an extension portion of the surgical guide extending away from the dental arch contacts the roof of the oral cavity of the subject, and the guide hole is thereby automatically placed over the entrance to the greater palatine foramen of the subject.

For some applications, the surgical guide is generated based on data from both a CT scan and an intra-oral scan. For such applications, an intra-oral scan of the upper palate, teeth, and/or gums of the subject is performed in addition to the CT scan, and the data from both scans are registered for preparation of the surgical guide. Alternatively, the surgical guide is initially generated based on data from an intra-oral scan only, and subsequently CT data are used for preparing the guide hole in the surgical guide.

Thus, in accordance with some applications of the present invention, the surgical guide is configured to guide an operating physician to the location of the greater palatine foramen of the subject, to facilitate advancement of the neural stimulator implant therethrough by injecting the implant into the canal. Additionally, the guide hole in the surgical guide facilitates penetration of the mucosa at an appropriate angle for entrance into the greater palatine foramen at an angle suitable for advancement of the neural stimulator implant through the canal. Further additionally, the CT data in combination with the surgical guide provides the operating physician with information regarding the anatomical structure of the greater palatine canal, thereby facilitating navigation and advancement of the implantable neural stimulator coupled to the steerable delivery guide through the canal. Thus, in accordance with some applications, the surgical guide in combination with the CT data, guides the passing through oral mucosa of the subject and navigation of the neural stimulator implant within a complex anatomical structure. Additionally, but not necessarily, the surgical guide provides guidance for drilling at a predetermined depth into the jaw bone.

The surgical guide typically allows for use of the neural stimulator implant by facilitating precise and safe implant deployment at the SPG, even by a less-skilled surgeon. Similarly, in general, the surgical guide allows a less-skilled surgeon to access the SPG in a safe and precise manner (even in the absence of implanting a neural stimulator implant).

For some applications, the delivery guide is configured to facilitate delivery of the neural stimulator to the SPG site without the need for the physician to consider a navigation map of the greater palatine canal. For some such applications, CT data regarding the anatomical structure of the greater palatine canal is used to create (typically by 3D printing) a curved guide groove surface on a portion of the delivery guide. When the neural stimulator is mounted on a distal end of the delivery guide, it is advanced distally in the canal by advancement of a slide-bar of the delivery guide. At the same time, a guiding pin which is disposed within the curved guide groove is advanced within the groove, causing rotation of the slide-bar with respect to the delivery guide, thereby steering the neural stimulator in the greater palatine canal.

For some applications, a shape-sensing optical fiber optically couplable to an optical fiber shape-sensing system is provided. The shape-sensing optical fiber typically is advanced by a delivery tool, e.g., a trocar, through the palatine canal and a shape of the canal is assessed using the shape-sensing optical fiber. The shape-sensing optical fiber is then removed from the canal. Subsequently, the neural stimulator implant is advanced and navigated through the palatine canal based on the assessing of the shape of the canal by the shape-sensing optical fiber.

For other applications, the neural stimulator implant additionally comprises the shape-sensing optical fiber optically couplable to an optical fiber shape-sensing system. The shape-sensing optical fiber is configured to change shape during delivery of the implant to the SPG through the greater palatine canal, to facilitate advancement and navigation of the implant through the canal.

Additionally or alternatively, navigation of the neural stimulator implant to the SPG through the greater palatine canal is facilitated by impedance-based navigation circuitry. Typically, the impedance-based navigation circuitry assesses a disposition, e.g., a location, a shape and/or an orientation, of the neural stimulator implant in the greater palatine canal.

For such applications, the implantable neural stimulator has an implant-impedance-sensing electrode, and impendence is measured between the implant-impedance-sensing electrode and an auxiliary-impedance-sensing electrode. Typically, the auxiliary-impedance-sensing electrode is disposed on an adhesive patch positioned on a face of the subject. Alternatively, the auxiliary-impedance-sensing electrode is disposed on the delivery tool.

The impedance-based navigation circuitry typically comprises a voltage generator configured to apply current between the implant-impedance-sensing and auxiliary-impedance-sensing electrodes through first and second wires electrically coupled respectively to the implant-impedance-sensing and auxiliary-impedance-sensing electrodes. The impedance-based navigation circuitry typically further comprises an impedance sensor configured to measure an impedance between the implant-impedance-sensing and auxiliary-impedance-sensing electrodes based on the applying of the current, and a disposition tracker configured to determine, based on the impedance measurements, e.g., a change in the measured impedance, a disposition of the implantable neural stimulator in the greater palatine canal.

In another application, at least one sensor coil is coupled to the implantable neural stimulator, and at least one transmitter coil, is disposed outside the subject's body. The transmitter coil generates electromagnetic fields at each of a plurality of field strengths, which induce respective currents in the sensor coil. Control circuitry is coupled to the sensor coil, and comprises a current sensor coupled to the at least one sensor coil and configured (i) to determine which of the respective induced currents passes a predetermined threshold, and (ii) to generate a signal in response to determining that the predetermined threshold has been passed. A disposition tracker determines, based on the signal, a disposition of the implantable neural stimulator in the greater palatine canal.

The invention provides an apparatus as claimed in claim 1. Optional features are set out in the dependent claims.

### BRIEF DESCRIPTION OF THE DRAWINGS

Fig. 1 shows a system for delivery of a neural stimulator implant for electrical stimulation of a sphenopalatine ganglion (SPG) of a subject, in accordance with some applications of the present invention;
Fig. 2 is a schematic illustration of a delivery guide being advanced through a guide hole of an oral surgical guide, in accordance with some applications of the present invention;
Figs. 3A, 3B, 3C, 3D, 3E, 3F, 3G and 3H are schematic illustrations of various configurations of the surgical guide shaped to define a guide hole for locating the entrance to the greater palatine canal, in accordance with some applications of the present invention;
Figs. 4A, 4B, 4C, and 4D are schematic illustrations of the system for delivery of a neural stimulator implant for electrical stimulation of a sphenopalatine ganglion (SPG) of a subject, in accordance with some applications of the present invention;
Fig. 5 is a schematic illustration of the neural stimulator implant for electrical stimulation of a sphenopalatine ganglion (SPG) of a subject, in accordance with some applications of the present invention;
Fig. 6 is a schematic illustration of a tool for facilitating delivery of a neural stimulator implant for electrical stimulation of a sphenopalatine ganglion (SPG) of a subject, in accordance with some applications of the present invention;
Fig. 7 is a schematic illustration of a tool for facilitating delivery of a neural stimulator implant for electrical stimulation of a sphenopalatine ganglion (SPG) of a subject, in accordance with some applications of the present invention;
Fig. 8 is a schematic illustration of a neural stimulator implant mounted onto a tool for facilitating delivery thereof for electrical stimulation of a sphenopalatine ganglion (SPG) of a subject, in accordance with some applications of the present invention;
Figs. 9A and 9B are schematic illustrations of the neural stimulator implant, in accordance with some applications of the present invention;
Fig. 10 is a schematic illustration of the neural stimulator implant, in accordance with some applications of the present invention;
Fig. 11 is a schematic illustration of the neural stimulator implant having a bent distal end, in accordance with some applications of the present invention;
Figs. 12A, 12B and 12C are schematic illustrations of a tool comprising a guiding groove for facilitating delivery of a neural stimulator implant for electrical stimulation of a sphenopalatine ganglion (SPG) of a subject, in accordance with some applications of the present invention;
Fig. 13 is a block diagram showing steps for preparation of a surgical guide, in accordance with some applications of the present invention;
Figs. 14A and 14B are schematic illustrations of a neural stimulator implant and an optical fiber, mounted onto a delivery tool for facilitating delivery of the implant, for electrical stimulation of a sphenopalatine ganglion (SPG) of a subject, in accordance with some applications of the present invention;
Figs. 15A, 15B, 16 and 17 are schematic illustrations of a neural stimulator implant and an optical fiber;
Figs. 18A, 18B and 18C are schematic illustrations of apparatus comprising a neural stimulator, an optical fiber and a tool;
Fig. 19 is a flow chart showing a method use;
Fig. 20 is a flow chart showing a method use;
Fig. 21 is a schematic illustration of apparatus comprising an optical fiber and a delivery tool;
Figs. 22A and 22B are schematic illustrations of an impedance-based navigation system for facilitating navigation of the neural stimulator in accordance with some applications of the present invention;
Fig. 23 is a flow chart showing a method for use;
Fig. 24 is a schematic illustration of a delivery tool for facilitating delivery of the neural stimulator implant, the delivery tool comprising an electrical force sensor for facilitating navigation of implant to a sphenopalatine ganglion (SPG) of a subject; and
Fig. 25 is a schematic illustration of an electromagnetic-based sensing system used to determine a disposition of the implantable neural stimulator in a greater palatine canal.

### DETAILED DESCRIPTION OF APPLICATIONS

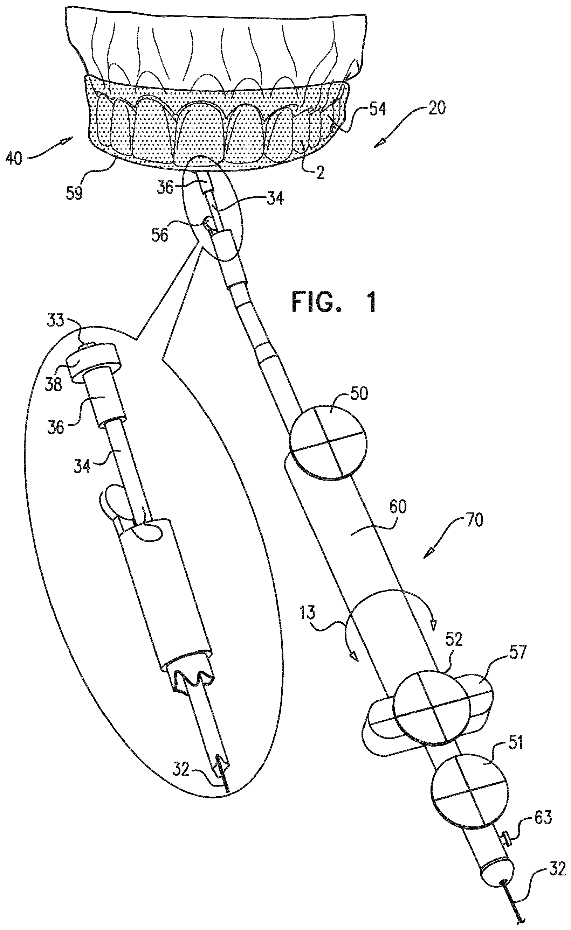

Reference is made to Fig. 1, which is a schematic illustration of a system 20 for delivery of a neural stimulator implant 32 for electrical stimulation of a sphenopalatine ganglion (SPG) of a subject, in accordance with some applications of the present invention. Typically, system 20 includes neural stimulator implant 32, steerable delivery guide 34, and an oral surgical guide 40.

Typically, neural stimulator implant 32 is configured to be passed through a greater palatine foramen of the hard palate of the oral cavity of the subject, into a greater palatine canal, such that the neural stimulator implant is brought into a vicinity of a sphenopalatine ganglion (SPG). For some applications, the implant is an elongated, flexible implant having an unconstrained shape and configured to conform to the anatomical structure of the greater palatine canal, for advancement therethrough. For some applications, the implant comprises at least one electrode, e.g., a wire electrode, for stimulation of the SPG. Typically, implant 32 is shaped to define a curved or bent distal end, which facilitates steering of the implant during the advancing of the implant in the canal. (For the purposes of the specification and claims of the present patent application, the terms "curved" or "bent" with respect to the distal end of the implant are to be understood as interchangeable.) Typically, following the advancing of the implant and deployment thereof in the vicinity of the SPG, for some subjects, the distal end of the implant is constrained and substantially not curved due to the anatomy of the canal, which is generally straight in the vicinity of the SPG in these subjects. For other subjects, the canal is curved in the vicinity of the SPG, and thus the distal end of the implant is curved at its implantation site in the vicinity of the SPG.

For some applications, neural stimulator implant 32 is coupled to steerable delivery guide 34. Implant 32 is configured to be passed through guide 34, such that both implant 32 and guide 34 are advanced through the greater palatine foramen into the greater palatine canal, and implant 32 is brought into a vicinity of a sphenopalatine ganglion (SPG). Steerable delivery guide 34 is retracted after placement of implant 32.

Fig. 1 shows an exploded view of neural stimulator implant 32 passed through delivery guide 34. Delivery guide 34 is typically less flexible than neural stimulator implant 32, and thereby facilitates smooth passage of the implant through the greater palatine canal and proper delivery of implant 32 to the SPG.

For some applications, a distal end 33 of steerable delivery guide 34 is configured to puncture oral mucosa of the subject, allowing neural stimulator implant 32 to be passed through the palate in a minimally-invasive procedure, without requiring a prior surgical incision in the mucosa. Typically, the distal end of the steerable delivery guide is also configured to be passed through the greater palatine foramen into the greater palatine canal. The delivery guide is steered in the canal in order to deliver the neural stimulator implant to the SPG. For some applications, neural stimulator implant 32 is configured to puncture or otherwise create an opening in the oral mucosa of the subject. Following insertion of implant 32 into the mucosa, the surgeon may optionally seal the puncture site by applying pressure to the puncture site in order to facilitate self-healing of the hole, e.g., by keeping a finger on the puncture site.

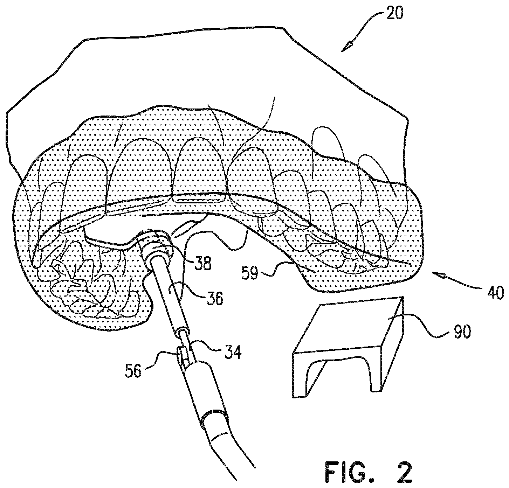

Fig. 1 additionally shows surgical guide 40 (represented by the dotted structure) placed on teeth 2 of a dental arch 54 of the subject. (It is to be understood that for subjects without teeth, guide 40 is placed on the gums.) Surgical guide 40 is generated based on CT data of the subject and typically serves as a guide for locating the entrance to the greater palatine canal through the greater palatine foramen of the hard palate. Surgical guide 40 comprises an arch portion 59 configured for placement on dental arch 54, and an extension portion 58 (shown in Figs. 2-3) that extends away from the arch portion. The extension portion is shaped to define a guide hole 6 (shown in Figs. 2-3), which provides an operating physician with the location and preferred entry angle to the greater palatine foramen. Typically the location and angle of the entrance to the canal, as well as the length of the canal, varies among the population. Therefore, surgical guide 40 allows safe and accurate entry into the canal, and navigation therethrough, in accordance with the subject's anatomy, based on the CT data. Surgical guide 40 additionally inhibits excessive insertion of implant 32 into the canal.

For some applications, a distal end 38 of an angular guide 36 is placed on extension portion 58 of surgical guide 40 to facilitate advancement of delivery guide 34 through guide hole 6 in surgical guide 40. Typically, distal end 38 plugs into hole 6, such that angular guide 36 facilitates advancement of delivery guide 34 into hole 6 at the preferred angle, based on the CT data. When angular guide 36 is locked properly in place with respect to surgical guide 40, delivery guide 34 is released by turning knob 63 in order to allow advancement of guide 34 through guide hole 6. A tool 70 is configured to direct advancement of guide 34 through guide hole 6 and subsequently through the greater palatine foramen into the greater palatine canal. Handle 60 of tool 70 is steered and/or advanced, in order to direct motion of steerable delivery guide 34.

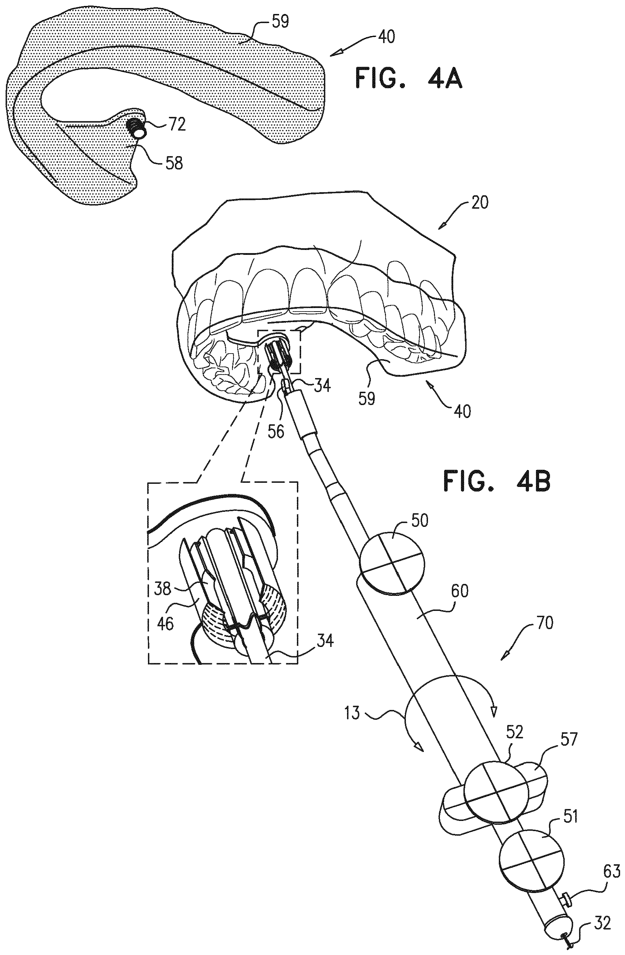

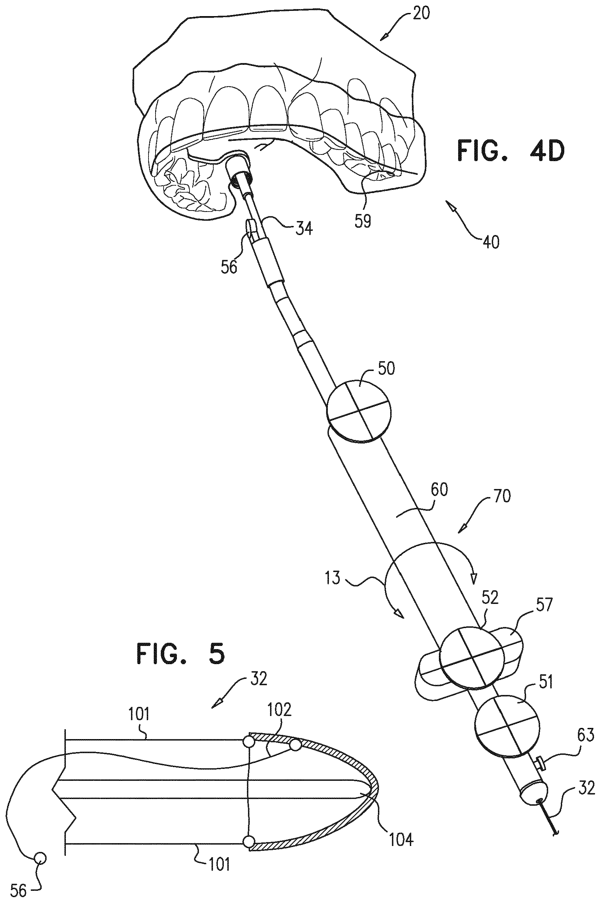

Typically, the passage of implant 32 and delivery guide 34 into the greater palatine canal is facilitated by image-guided surgical techniques, e.g., using optical fiducial markers 50, 51 and 52 on tool 70 (and/or fiducial markers on guide 34). For some applications, an image-guided surgery processor utilizes location data derived from markers 50, 51 and 52, in combination with fiducial markers on the subject (e.g., placed on the teeth, face or a head of the subject) in order to register the pre-operative CT data with the current position of the tool and thereby facilitate steering and advancement of steerable delivery guide 34 through the greater palatine canal. Alternatively or additionally, the image-guided surgery processor utilizes location data derived from markers 50, 51 and 52 in combination with registration data obtained by (a) contacting a tool with a fiducial marker to multiple spots on the subject's head that can also be identified in the pre-operative CT image, and/or (b) visualizing markers 50, 51, and/or 52 when angular guide 36 is locked in place, for example, by plugging distal end 38 into guide hole 6 or by a locking mechanism (as described herein below with reference to Figs. 4A-C). For some applications, handle 60 comprises a linear and/or an angular encoder configured to facilitate recording of location data indicative of the current position and orientation of neural stimulator implant 32. It is noted that the fiducial markers described herein can be used both in order to identify locations in the subject's anatomy, and also as a reference marker, in order to continually allow the image-guided surgery processor to identify a current position of the subject's head, which can move.

Additionally, slide-bar 57 on tool 70 facilitates advancement of delivery guide 34 distally through guide hole 6. Typically, slide-bar 57 provides steering functionality for facilitating advancement of guide 34 into the greater palatine canal. Bar 57 is typically slidable with respect to handle 60. Advancement of slide-bar 57 with respect to handle 60 advances delivery guide 34 through the greater palatine canal. Additionally or alternatively, marker 50 comprises steering functionality and is rotated around a center thereof in order to steer guide 34 and neural stimulator implant 32 within the canal in order to deliver the neural stimulator implant to the SPG. Further additionally or alternatively, handle 60 is rotated as indicated by arrow 13, in order to advance and orientate steerable delivery guide 34 within the greater palatine canal.

For some applications, additional steering options are employed to allow control of the advancement of implant 32 within the canal. For example, using a joystick allows steering the implant in a left/right and up/down direction, as well as rotation around an axis.

Typically, the greater palatine canal is curved and multiple openings are naturally formed along the greater palatine canal. Therefore, proper steering of guide 34 within the canal generally ensures delivery of guide 34 and neural stimulator implant 32 to the SPG.

For some applications, surgical guide 40 is coupled to or used in association with a second arch portion (not shown). The second arch portion is typically configured for placement on a lower dental arch of the subject. The second arch portion typically stabilizes upper arch portion 59, by pressing portion 59 against the upper teeth and palate. Additionally or alternatively, a stabilizing element 90 is placed between the lower and upper dental arches of the subject, and facilitates the squeezing of arch portion 59 against the upper teeth and palate.

Reference is made to Fig. 2, which is a schematic illustration of steerable delivery guide 34 being steered and advanced through guide hole 6 of surgical guide 40, in accordance with some applications of the present invention. Surgical guide 40 comprises arch portion 59 configured for placement on dental arch 54 and extension portion 58 which is shaped to define guide hole 6. Extension portion 58 contacts the roof of the oral cavity of the subject, and guide hole 6 is thereby automatically placed over the entrance to the greater palatine foramen of the subject.

Thus, in accordance with some applications of the present invention, surgical guide 40 is configured to guide an operating physician to the location of the greater palatine foramen of the subject, to facilitate advancement of guide 34 therethrough. Additionally, guide hole 6 in the surgical guide facilitates penetration of the mucosa at an appropriate angle for entrance into the greater palatine foramen at an angle suitable for advancement of guide 34 through the canal. Further additionally, the CT data in combination with the surgical guide provide the operating physician with information regarding the anatomical structure of the greater palatine canal, thereby facilitating navigation and advancement of neural stimulator implant 32 coupled to steerable delivery guide 34 through the canal.

For some applications, a length of a portion (e.g., a protrusion) of surgical guide 40 controls the degree to which neural stimulator implant 32 may be inserted into the canal. In other words, the length of the portion (e.g., protrusion) inhibits excessive insertion of implant 32 into the canal, and is designed such that the length of the portion controls the distance to which neural stimulator implant 32 is advanced in the canal. Thus, advancement of implant 32 is terminated based on contact of a portion of the delivery guide with the portion of the surgical guide that has a length corresponding to the location of the SPG. The location of the SPG and the shape of the canal are assessed by a pre-operative CT scan, and surgical guide 40 is typically configured to facilitate insertion of neural stimulator implant 32 to the location of the SPG based on the CT scan data. The shorter the length of the portion (e.g., the protrusion) of surgical guide 40, the farther neural stimulator implant 32 is advanced in the canal.

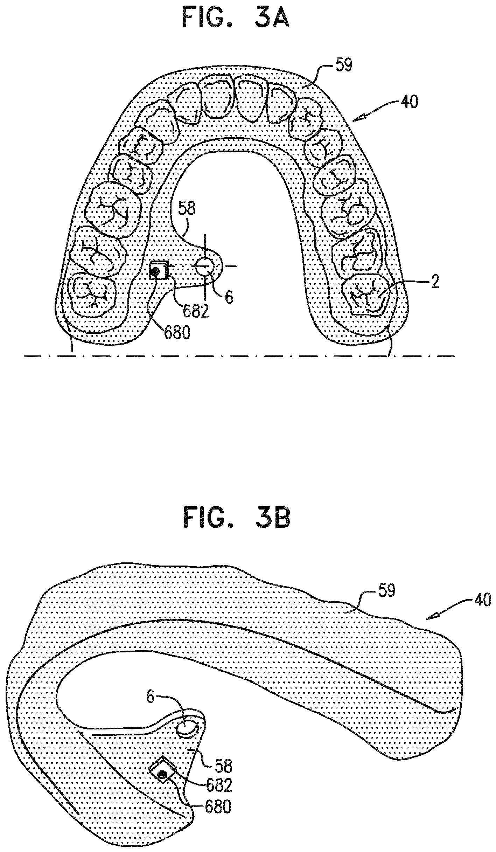

Figs. 3A-B are schematic illustrations of surgical guide 40 comprising arch portion 59 configured for placement on teeth 2 of a subject, or on gums of the subject, in accordance with some applications of the present invention. Extension portion 58 extends, lingually and in a superior direction, away from arch portion 59 and is placed in contact with the roof of the oral cavity of the subject. Extension portion 58 is shaped to define guide hole 6, which is automatically placed over the entrance to the greater palatine foramen when surgical guide 40 is placed on teeth 2, or gums, of the subject. For some applications, an adhesive, e.g., glue, is used to secure guide 40 to the teeth or gums of the subject.

Typically the location of the greater palatine foramen varies among the population. For example, in some subjects the greater palatine foramen is associated with the upper third molar tooth. In other subjects, the greater palatine foramen is associated with the second molar or between the second and third molar. It is noted that the location of guide hole 6 is shown in the figures by way of illustration and not limitation. It is understood that the location of guide hole 6 is set based on the location of the greater palatine foramen of each particular subject. Surgical guide 40 is typically custom-made based on a CT scan of the subject, such that guide hole 6 is placed over the greater palatine foramen of each individual subject, in order to guide the physician to the correct location.

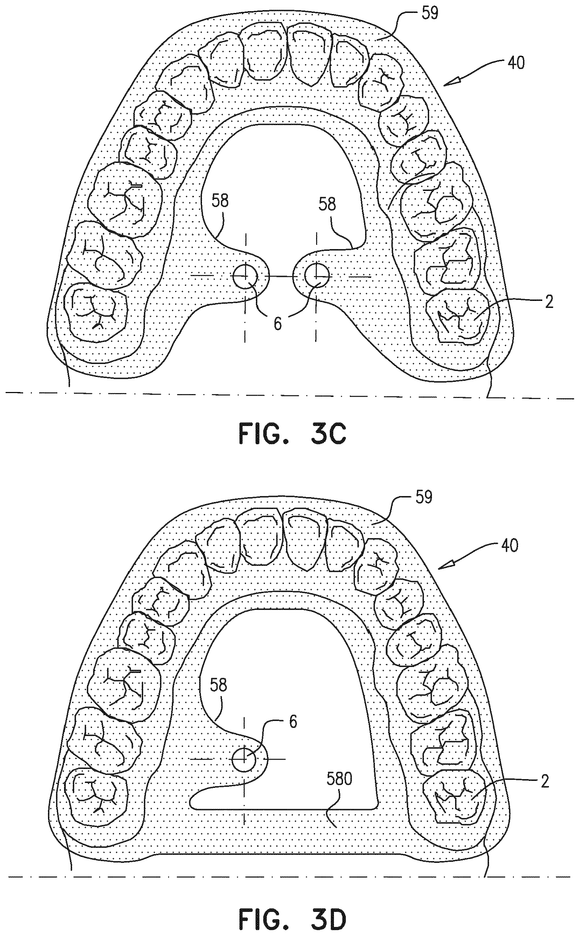

Reference is now made to Fig. 3C. For some applications, surgical guide 40 comprises a second extension portion 58 located contralateral to extension portion 58, for bilateral electrical stimulation of the right and left SPG (e.g., for treatment of vascular dementia).

For some applications, surgical guide 40 is fabricated by three-dimensional (3D) printing. For some applications, for example in order to reduce fabrication time of guide 40, guide 40 comprises a non-patient-customized portion (e.g., made of metal, molded plastic, a generic part made of a 3D-printed material, and/or a combination of materials (e.g., a combination of plastic and metal), and a patient-customized portion (e.g., produced by 3D printing especially for the patient).

Alternatively, surgical guide 40 is manufactured by molding a pliable material, such as a thermoplastic sheet, and drilling guide hole 6 with a drill. (After the molding, a suitable process is used to make the pliable material generally rigid, e.g., by heat treatment or ultraviolet curing.)

Typically, the drill has markers (e.g., RF coils, or optical markers) in order to ensure drilling of guide hole 6 in a proper location corresponding to the greater palatine foramen. Typically, prior to drilling of the hole, the unfinished surgical guide is placed on teeth or gums of the subject and CT data of the oral cavity is acquired. Subsequently, the surgical guide is removed from the subject's mouth. Using a processor, the CT data of the oral cavity with the surgical guide is received and is used to determine a desired position of the drill. Directional and orientational guidance for performing the drilling is generated using the one or more markers on the drill. Subsequently, the processor guides drilling of the hole in the surgical guide at a site on the surgical guide which corresponds to the greater palatine foramen of the subject.

Reference is now made to Fig. 3D. For some applications, surgical guide 40 additionally comprises a support element 580A. Support element 580A typically extends from a first side of surgical guide 40 to a second side of guide 40 (e.g., an opposite side, e.g., extending from the left to the right side). Support element 580A typically extends from a left side of arch portion 59 to a right side of arch portion 59, posterior to a canine region of oral surgical guide 40. Support element 580A typically enhances rigidity of guide 40 and inhibits movement of surgical guide 40 when pressure is applied to guide 40 during insertion of angular guide 36 through hole 6. Additionally or alternatively, surgical guide 40 is thickened in order to add to rigidity thereto, optionally in the absence of support element 580A.

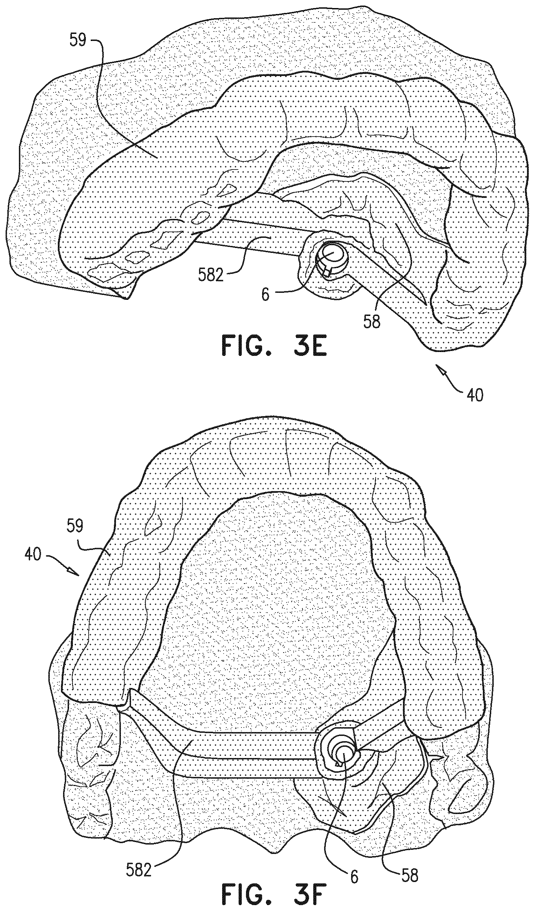

Reference is now made to Figs. 3E and 3F. For some applications, surgical guide 40 comprises a support element 582. Support element 582 typically extends from extension portion 58 to a right side of arch portion 59, posterior to a canine region of oral surgical guide 40. Support element 582 is generally the same as support element 580A and enhances rigidity of guide 40 and inhibits movement of surgical guide 40 when pressure is applied to guide 40 during insertion of (for example) angular guide 36 through hole 6.

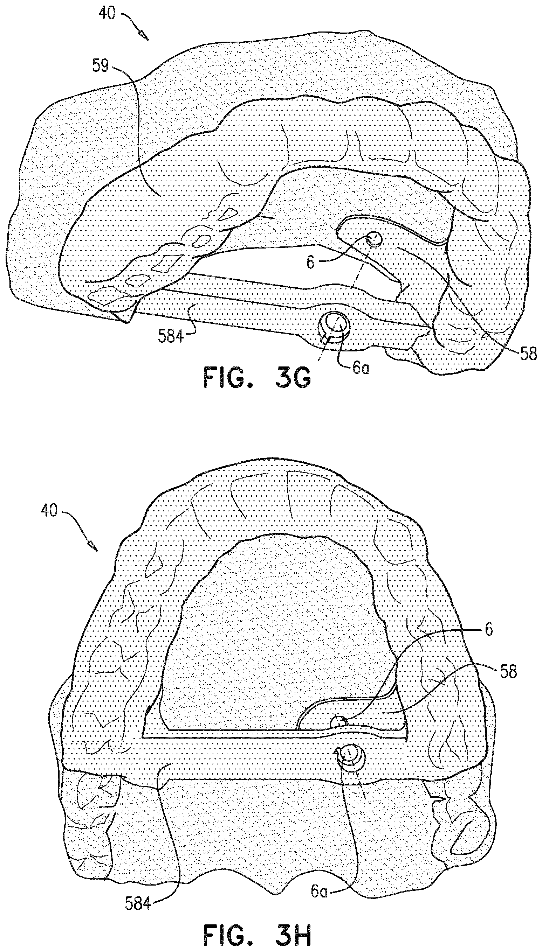

Reference is now made to Figs. 3G and 3H. For some applications, surgical guide 40 comprises a support element 584. Support element 584 typically extends from a left side of arch portion 59 to a right side of arch portion 59, posterior to a canine region of oral surgical guide 40 and inferior and in a lingual direction with respect to arch 59 (i.e., more at the level of the teeth than at the level of the palate). For such applications, support element 584 is shaped to define a guide hole 6a which is aligned with guide hole 6 in extension portion 58, allowing access to guide hole 6 through guide hole 6a. Optionally but not necessarily hole 6a is shaped to define a funnel. Support element 584 enhances rigidity of guide 40 and inhibits movement of surgical guide 40 when pressure is applied to guide 40 during insertion of angular guide 36 through hole 6.

Reference is made to Figs. 4A-C, which are schematic illustrations of dental arch portion 59, comprising a locking mechanism 94, in accordance with some applications of the present invention. Locking mechanism 94 is configured to lock tool 70 and angular guide 36 in place with respect to surgical guide 40, such that delivery guide 34 and implant 32 are advanced accurately through guide hole 6. Generally, locking mechanism 94 comprises (a) a projecting portion of surgical guide 40 which is typically shaped to provide a screw thread on an outer surface of projection 72, and (b) a screw thread on an inner surface of the locking portion on tool 70. The screw threads on projection 72 and on tool 70 engage each other, thereby locking the projection to the tool.

Fig. 4A shows surgical guide 40 comprising arch portion 59 and extension portion 58. For some applications, extension portion 58 further comprises projection 72, which protrudes away from extension portion 58. Projection 72 is typically shaped to define the screw thread profile described hereinabove, on an outer surface of the protrusion (as shown). (Alternatively, the screw-thread is on the inner surface of the projection.)

Reference is made to Fig. 4B. For some applications, angular guide 36, which is mounted to tool 70, comprises locking portion 46 which is shaped to define a screw thread (described hereinabove), configured to engage projection 72 on surgical guide 40. Locking portion 46 is typically rotated in order to lock locking portion 46 to projection 72, thereby restricting motion of delivery guide 34.

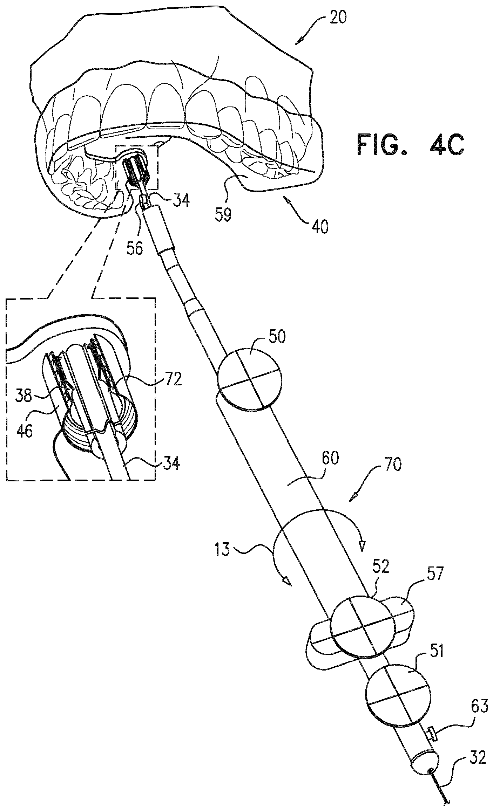

Fig. 4C shows locking mechanism 94 in a locked state thereof. It is to be noted that surgical guide 40 is shaped to define a screw-shaped projection 72 by way of illustration and not limitation. In general, surgical guide 40 may comprise a first coupling, and guide 36 and/or tool 70 may comprise a second coupling. The first coupling may comprise a male coupling while the second coupling may comprise a female coupling, or vice versa.

It is noted that locking mechanism 94 is described by way of illustration and not limitation. For some applications, tool 70 and angular guide 36 are locked in place with respect to surgical guide 40 by plugging distal end 38 into guide hole 6. For example, locking of tool 70 with respect to surgical guide 40 is allowed when angular guide 36 is plugged into guide hole 6 at an appropriate angle and/or a particular orientation (e.g., via a fin extending at 12 o'clock that fits into a corresponding slot on surgical guide 40).

Reference is made to Fig. 5, which is a schematic illustration of an example of neural stimulator implant 32 for electrical stimulation of a sphenopalatine ganglion (SPG) of the subject, in accordance with some applications of the present invention.

Neural stimulator implant 32 is typically 0.5-1.5 mm in diameter, e.g., 1 mm. Thus, advancement of implant 32 typically does not require dilation of the greater palatine canal. Alternatively, placement of implant 32 includes pre-dilation of the greater palatine canal.

For some applications, neural stimulator implant 32 is electrically coupled to circuitry 56 which is adapted to be placed outside the greater palatine canal, e.g., the circuitry may be positioned submucosally in the oral cavity. For other applications, circuitry 56 is adapted for insertion into the oral mucosa of the subject. Following insertion of electronic circuitry 56 into the mucosa, the surgeon may seal the puncture site by applying pressure to the puncture site in order to facilitate self-healing of the hole, e.g., by keeping a finger on the puncture site. Typically, neural stimulator implant 32 itself is configured for puncturing the oral mucosa.

For some applications, electronic circuitry 56 is advanced along an exterior of delivery guide 34 (as shown), until circuitry 56 is inserted into the mucosa.

As shown in Fig. 5, implant 32 typically comprises at least two steering wires 101 configured to facilitate steering of implant 32 within the greater palatine canal. Additionally, implant 32 comprises a stimulation wire 102 coupled to an electrode 106, for electrical stimulation of the sphenopalatine ganglion (SPG) of the subject, once implant 32 is delivered to the vicinity of the SPG.

Typically, the delivery apparatus comprises a pusher 104 disposed within delivery guide 34 (Fig. 1), which is configured to advance implant 32 within the greater palatine canal, e.g., by pushing an inner surface of electrode 106.

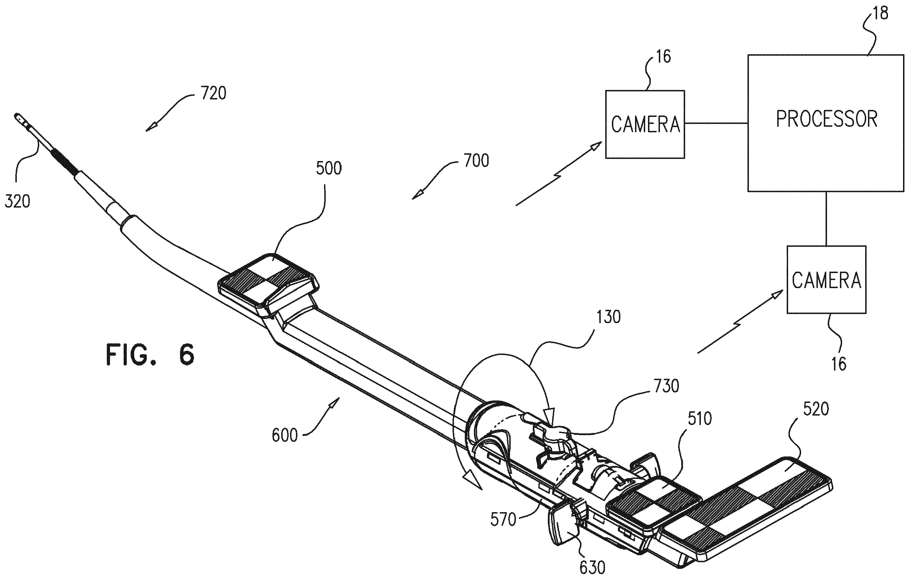

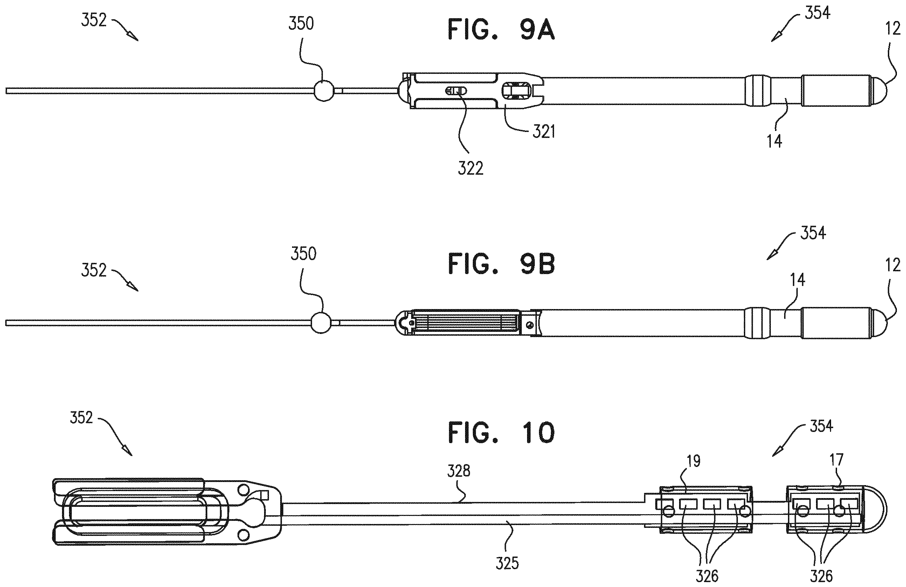

Reference is made to Fig. 6, which is a schematic illustration of a delivery tool 700 for facilitating delivery of a neural stimulator implant 320 (described hereinbelow with reference to Figs. 9A-B and 10) to a sphenopalatine ganglion (SPG) of a subject, for electrical stimulation of the SPG, in accordance with some applications of the present invention.

Tool 700 is typically used in combination with surgical guide 40 (described herein with reference to Figs. 3A-C) and directs advancement of the neural stimulator implant through guide hole 6 in surgical guide 40 and subsequently through the greater palatine foramen into the greater palatine canal.

Tool 700 typically comprises a handle 600 and a distal tip portion 720. In general, prior to use, the neural stimulator implant is mounted in distal tip portion 720. Fig. 6 shows the implant partially protruding from tip portion 720, as it appears after it has been initially advanced into the greater palatine canal. (For clarity of illustration, surgical guide 40 and anatomy are not shown.) Overall, tool 700 facilitates advancement of the implant toward the sphenopalatine ganglion (SPG) of a subject.

Typically, distal tip portion 720 plugs into surgical guide 40 to facilitate accurate advancement of neural stimulator implant 320 through guide hole 6 in surgical guide 40. Handle 600 comprises a slide-bar 570, which is slidable with respect to handle 600. Slide-bar 570 is typically locked in place, until it is released by a release mechanism 730 (e.g., by turning a knob on handle 600), in order to allow advancement of the neural stimulator implant through the guide hole and into the greater palatine canal.

An operating physician typically slides slide-bar 570 along handle 600 in order to advance implant 320 out of tool 700 and distally through guide hole 6. Additionally, slide-bar 570 provides steering functionality for facilitating orientation of the implant in the greater palatine canal. Advancement of slide-bar 570 with respect to handle 600 advances the implant through the canal.

For some applications, slide-bar 570 is rotated as indicated by arrow 130, in order to orient implant 320 within the greater palatine canal. Typically, a distal-most portion of implant 320 is oriented at a non-zero angle with respect to a longitudinal axis of the implant, such that the implant may be steered in the palatine canal in an analogous fashion to that in which a steerable guidewire is steered in the vasculature of a subject.

For some applications, the passage of implant 320 into the greater palatine canal is facilitated by image-guided surgical techniques, e.g., using optical fiducial markers 500, 510 and 520 on tool 700. Two or more cameras 16 are used to image markers 500, 510, and 520. An image-guided surgery processor 18 coupled to receive the image data from the cameras utilizes location data derived from markers 500, 510 and 520, in combination with fiducial markers on the subject (e.g., placed on surgical guide 40, or the teeth, face or a head of the subject) to register pre-operative CT data (showing bony structures in general and the greater palatine canal in particular) with the current position of the tool and thereby facilitate steering and advancement of implant 320 through the greater palatine canal.

Alternatively or additionally, the image-guided surgery processor utilizes location data derived from markers 500, 510 and 520 in combination with registration data obtained by (a) contacting a tool with a fiducial marker to multiple spots on the subject's head that can also be identified in the pre-operative CT image, and/or (b) visualizing markers 500, 510, and/or 520 when distal tip portion 720 is secured to surgical guide 40.

For some applications (in addition to or instead of using markers 500, 510, and 520), handle 600 comprises a linear and/or an angular encoder configured to facilitate recording of location data indicative of the current position and orientation of neural stimulator implant 320.

It is noted that processor 18 is typically a programmed digital computing device comprising a central processing unit (CPU), random access memory (RAM), non-volatile secondary storage, such as a hard drive or CD ROM drive, network interfaces, and/or peripheral devices. Program code, including software programs, and/or data are loaded into the RAM for execution and processing by the CPU and results are generated for display, output, transmittal, or storage, as is known in the art. Such program code and/or data, when provided to the processor, produce a machine or special-purpose computer, configured to perform the tasks described herein.

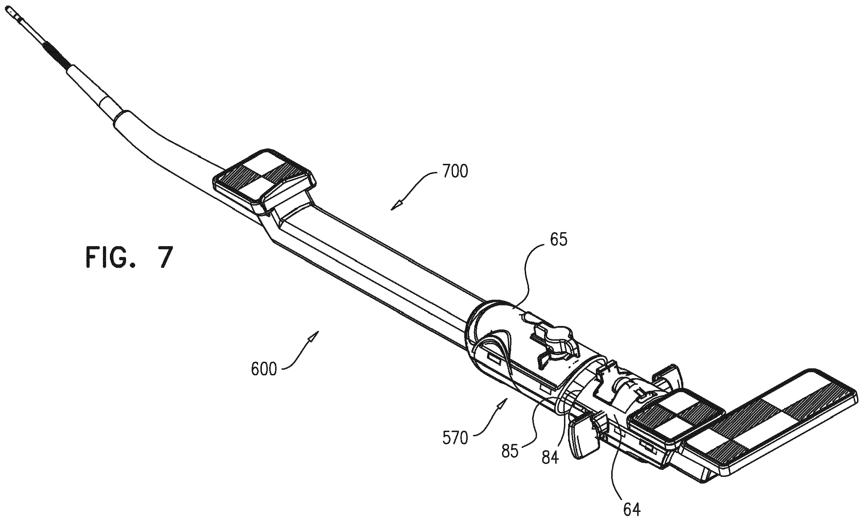

Reference is made to Fig. 7, which is a schematic illustration of delivery tool 700, generally as described herein with reference to Fig. 6. For some applications, slide-bar 570 of handle 600 comprises a distal portion 65 and a proximal portion 64, which are held connected to each other by first and second magnetic elements 85 and 84 coupled to the proximal and distal portion of slide-bar 570 and magnetically coupled to each other. Proximal portion 64 of slide-bar 570 is coupled to implant 320 such that distal advancement of proximal portion 64 of the slide-bar produces distal advancement of the implant. Typically, the physician advances the slide-bar by gripping distal portion 65 and applying a distally-directed force thereto, such that the magnetic coupling causes proximal portion 64 to advance distally, and thereby cause distal advancement of implant 320. If the force applied to distal portion 65 of slide-bar 570 in a distal direction exceeds a threshold (e.g., due to advancement of the implant being impeded), this typically breaks the coupling between the first and second magnetic elements, thereby discontinuing advancement of implant 320 and alerting the operating physician to an issue relating to the proper placement of implant 320.

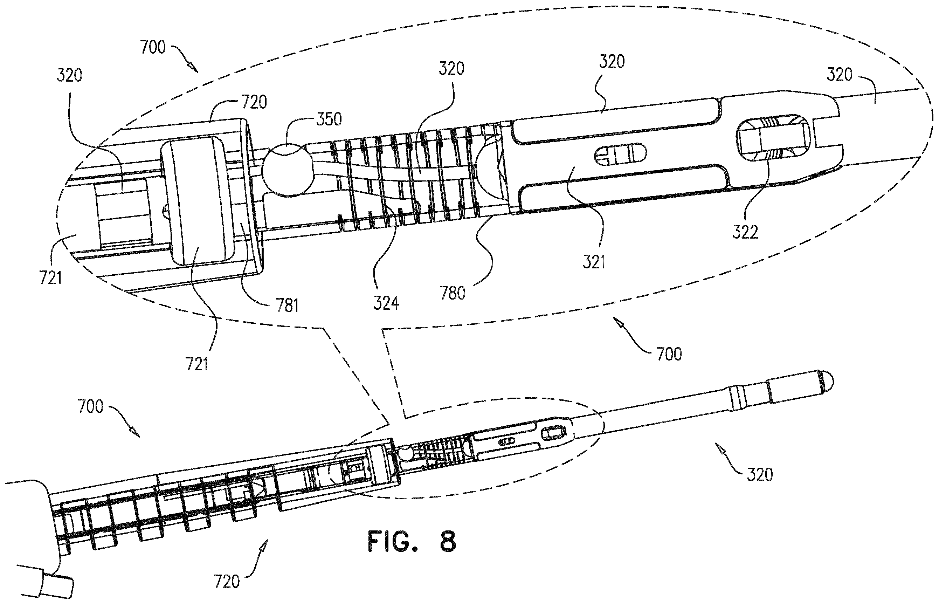

Reference is made to Fig. 8, which is a schematic illustration of neural stimulator implant 320 extending from distal tip portion 720 of tool 700, in accordance with some applications of the present invention. (Other components of tool 700 are labeled 721 in Fig. 8). For some applications, tool 700 comprises at a distal portion thereof, a stainless steel tube 780 configured to engage a locking element 350 of implant 320. An engaging element 781 is configured to engage locking element 350 of implant 320 (shown in Fig. 8 as a ball by way of illustration and not limitation). Typically, activation of an implant-release mechanism 630 (e.g., by turning a knob as shown in Fig. 6) causes engaging element 781 to disengage from locking element 350, allowing all implantation apparatus in the greater palatine canal to be withdrawn, generally without dislodging implant 320 from its implantation location near the SPG.

Typically, tube 780 is shaped to define a series of slits 324 longitudinally aligned along tool 700, each slit disposed at an angular offset (e.g., a 180 degree offset as shown in Fig. 8, or alternatively at a 90 degree offset, not shown) from an adjacent one of the slits. The slits permit tube 780 to bend in a range of directions, e.g., in any direction, to facilitate advancement of the implant through the greater palatine canal.

Implant 320 is generally flexible but typically also comprises a rigid portion 321 which houses a receiving coil 322 configured to receive power from a remote power source to power implant 320.

Reference is now made to Figs. 9A-11, which are different views of implant 320, in accordance with some applications of the present invention. As shown, implant 320 comprises proximal 352 and distal 354 portions. Implant 320 is a generally flexible, elongate implant having electrodes (e.g. a dome electrode 12 and a second electrode 14) at the distal portion thereof and an unconstrained shape that is curved, i.e., bent, in a vicinity of the distal portion (e.g., proximal to electrode 14, or between electrodes 12 and 14). Figs. 9A-B and 10 show implant 320 in a straight configuration. Typically, following the advancing of the implant and deployment thereof in the vicinity of the SPG, distal portion 354 of the implant is constrained and shaped differently due to the anatomy of the canal compared to its unconstrained shape. For example, distal portion 354 may be generally straight in the vicinity of the SPG, based on the anatomy of some subjects, or distal portion 354 may be curved at its implantation site in the vicinity of the SPG.

Implant 320, in particular distal portion 354, is typically configured to puncture oral mucosa of the subject in order to allow advancement of implant 320 into the greater palatine canal. For some applications, implant 320 is not configured to puncture the oral mucosa, but instead a distal portion of tool 700 is configured to puncture oral mucosa.

It is noted that for some applications, implant 320 comprises two or more portions of electronic circuitry comprising multiple circuitry units 326, at discrete longitudinal sites along implant 320 (shown in Fig. 10). Typically, the electronic circuitry is divided into first and second portions 17 and 19, which are coupled respectively to proximal and distal sites of neural stimulator implant 320 that are flexibly coupled to each other. Division of the electronic circuitry into two or more portions typically facilitates smooth advancement of the implant in the canal.

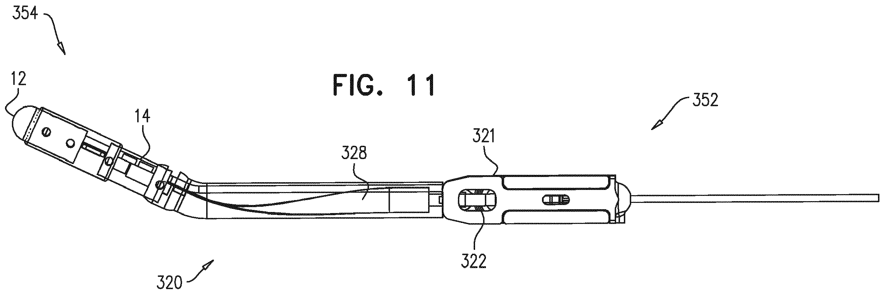

For some applications, a flexible, connecting element 328 (e.g., a flexible printed circuit board) extends along implant 320 and connects first and second portions 17 and 19 of the electronic circuitry. Alternatively or additionally, a structural element 325 able to withstand compressive forces associated with the implantation is used to convey distally-directed forces toward the distal end of implant 320. For example, this structural element may comprise nitinol (and for some applications is not used to convey electrical signals between the first and second portions of the electronic circuitry). Structural element 325 comprising nitinol typically has a trained natural curve, which enables steering of implant 320 by rotating the handle 600 of tool 700 (Fig. 6). The curve in element 325 could be as shown in Fig. 11, or between the two electrodes on distal portion 354, or within 15 mm of the very distal tip.

Fig. 11 shows neural stimulator implant 320 having a curved or bent distal end, as described hereinabove, in accordance with some applications of the present invention.

Reference is made to Figs. 1-12C and Figs. 14A-18C. For some applications, a surface shaped to define a guiding groove is generated (typically by a 3D printing process) based on CT data obtained by imaging the subject. Based on the CT data, the guiding groove is shaped in accordance with the subject's anatomy in order to guide the implant to the desired anatomical site, e.g., to guide steering of neural stimulator implants 32 and/or 320 through the greater palatine canal to the vicinity of the sphenopalatine ganglion (SPG).

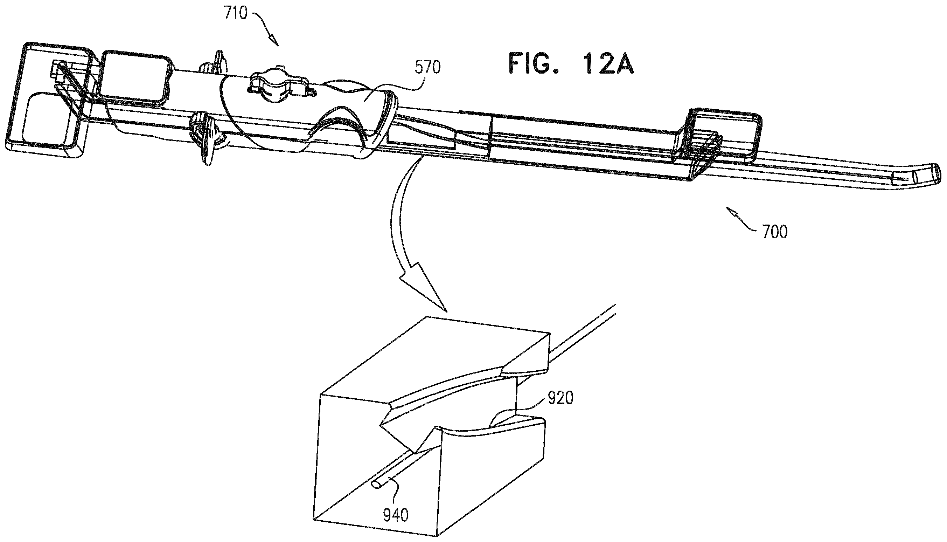

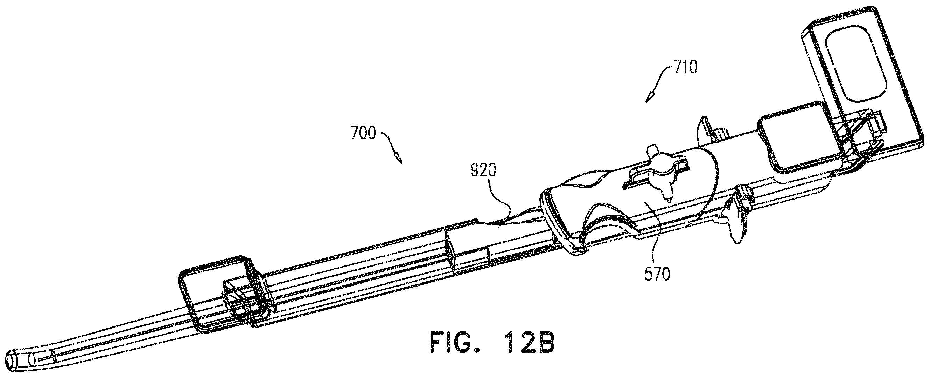

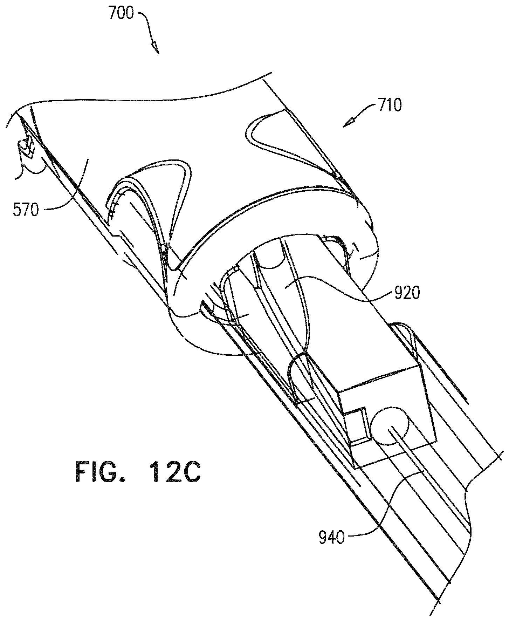

As shown in Fig. 12, a delivery tool, e.g., implantation tool 700, comprises a surface shaped to define a curved guide groove 920 at a proximal portion 710 of the delivery tool. Curved guide groove 920 is generated based on data obtained by imaging the anatomy of the subject, e.g., the greater palatine canal. A guiding pin 940 is typically disposed within curved guide groove 920, and is configured such that advancement of slide-bar 570 with respect to proximal portion 710 produces (1) relative motion of guiding pin 940 with respect to curved guide groove 920, and (2) rotation of slide-bar 570 with respect to a longitudinal axis of tool 700.

Typically, as the operating physician slides slide-bar 570 along handle 600, guide groove 920 correctly guides the pin, thereby steering the implant in the canal (i.e., by causing rotation of slide-bar 570 as indicated by arrow 130 in Fig. 6, at the correct point in the longitudinal advancement of slide-bar 570 to cause a corresponding steering of implants 32 and/or 320).

For some applications, guiding pin 940 is attached to delivery tool 700, e.g., guiding pin 940 is fixedly coupled to slide-bar 570 of tool 700. For such applications, the surface shaped to define curved guide groove 920 is a surface of tool 700. For other applications, guiding pin 940 is attached to tool 700 (e.g., to handle 600 and not to the slide-bar) and slide-bar 570 is shaped to define the surface with curved guide groove 920.

It is noted that these applications using the guiding groove may, but typically do not, utilize optical markers 500, 510, or 520, or many other electronic surgical guidance techniques known in the art. For some applications, the techniques described in this paragraph may be used for advancement of other tools, in sites other than the greater palatine canal (e.g., to facilitate endoscopic sinus surgery, or vascular catheterizations).

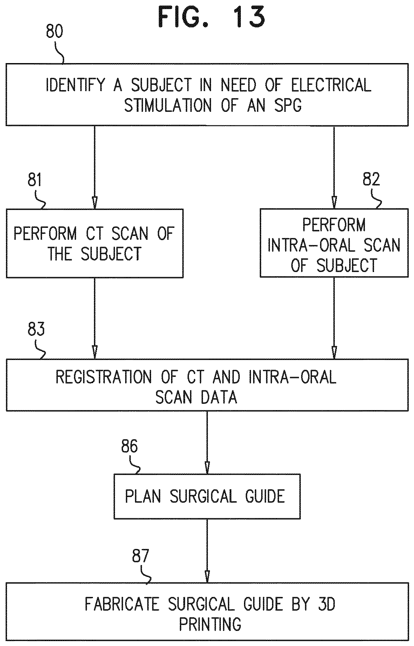

Reference is made to Figs. 3A-B and Fig. 13. For some applications, surgical guide 40 is generated based on data from both a CT scan and an intra-oral scan. For such applications, an intra-oral scan of the upper palate of the subject is performed in addition to the CT scan, and the data from both scans are registered for preparation of surgical guide 40.

An intra-oral scan typically contributes to fabrication of a better-fitting surgical guide 40 by providing high-resolution data of the upper palate including mapping of soft-tissue anatomy such as oral mucosa. For example, a portion of surgical guide 40 that corresponds to a surface of gum tissue of the subject is typically shaped in a curved manner that matches curvature of the gum tissue.

Thus, hole 6 is properly placed over the soft tissue that covers the greater palatine foramen. Having the surgical guide fit better over the oral mucosa typically facilitates optimal puncturing and penetration of the greater palatine foramen.

As described hereinabove, data obtained from the CT scan regarding bone and hard tissue of the subject, are typically used to determine the location and angle of implant insertion as well as guiding advancement of the implant to the SPG. Combining the data from both the CT scan and the intra-oral scan typically results in an enhanced surgical guide 40 in which both bone structure and the shape of soft tissue of the oral cavity are both reflected in surgical guide 40.

Fig. 13 is a block diagram showing steps of obtaining both CT scan data and intra-oral scan data for preparation of a surgical guide, in accordance with some applications of the present invention. Typically, in step 80, a subject in need of electrical stimulation of the SPG is identified. A CT scan and an intra-oral scan are then performed, as shown in steps 81 and 82. In step 83 the data from the CT and intra-oral scans are registered, and subsequently the surgical guide is planned and fabricated using the data from both the CT and intra-oral scanning (steps 86 and 87). As described hereinabove, surgical guide 40 is typically fabricated by three-dimensional printing techniques.

It is however noted that for some applications, surgical guide 40 is generated based on CT data only. Alternatively, for some applications, surgical guide 40 is generated based on intra-oral scan data only.

For some applications in which surgical guide 40 is generated based on intra-oral scan data only, a CT scan is performed after surgical guide 40 is generated. For example, CT data of the subject may be acquired while surgical guide 40 is disposed within the oral cavity, and registration of surgical guide 40 with respect to hard tissue of the anatomy may be performed using one or more markers affixed to surgical guide 40, and/or using features of the anatomy (e.g., teeth) that are imaged in the CT scan and in the intra-oral scan. The CT data typically guide the surgeon to drill a hole in surgical guide 40 at a site on the surgical guide that corresponds to the greater palatine foramen of the subject. For example, this drilling may be facilitated by markers on the drill, as described hereinabove. Subsequently, to drilling the hole, surgical guide 40 may be placed in the mouth and used to facilitate a procedure, as described hereinabove.

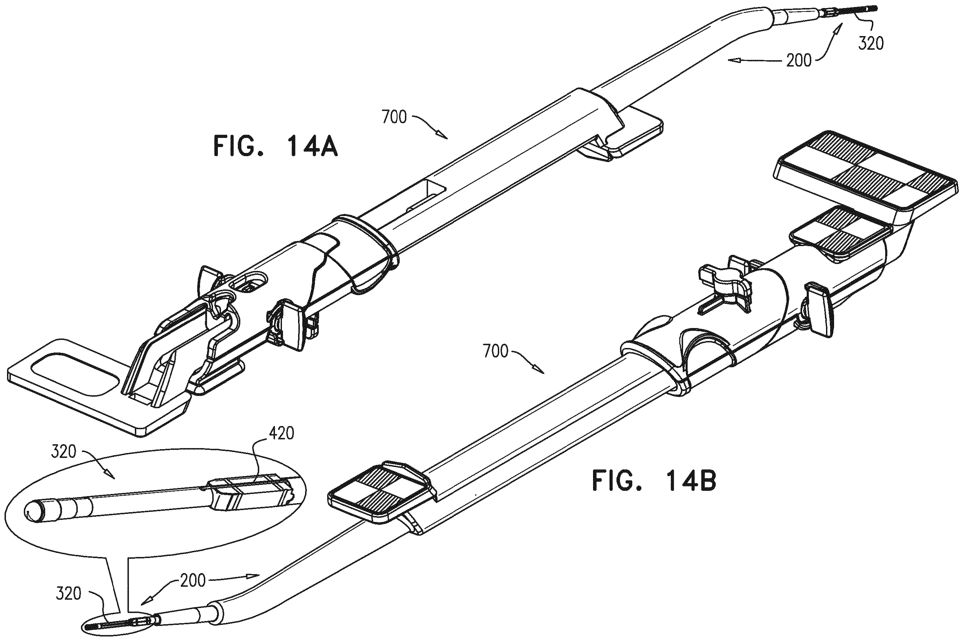

Reference is now made to Figs. 14A-B, which are schematic illustrations of apparatus 200 comprising neural stimulator implant 320 mounted onto delivery tool 700, in accordance with some applications of the present invention.

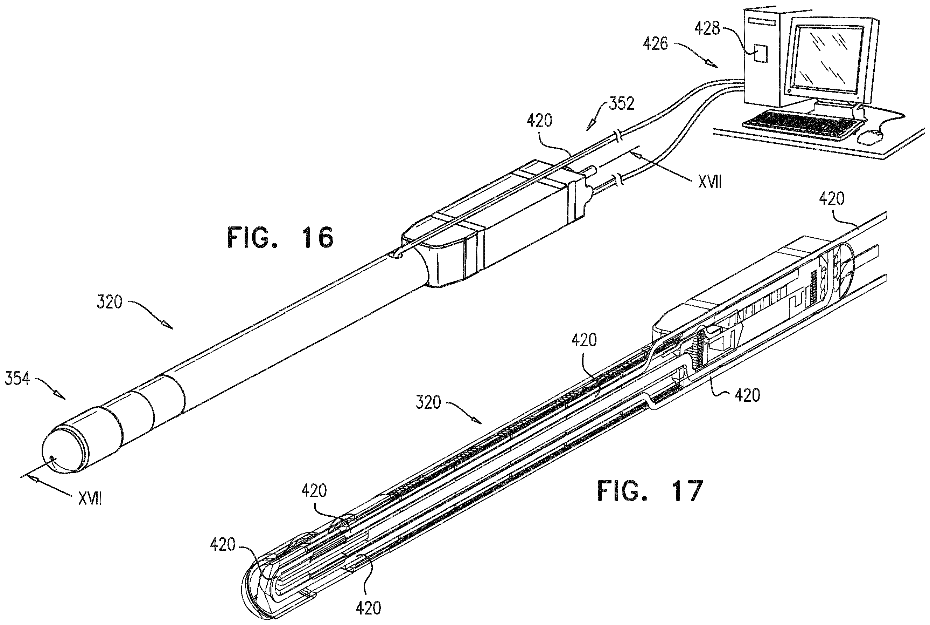

For applications shown in Figs. 14A-17, neural stimulator implant 320 additionally comprises a shape-sensing optical fiber 420. Optical fiber 420 is optically couplable to an optical fiber shape-sensing system 426 (Fig. 16, not to scale), and is configured to change shape during delivery of implant 320 to the SPG through the greater palatine canal of the subject.

In accordance with shape-sensing optical fiber technology, optical fiber 420 is typically used to monitor a dynamic three-dimensional shape of a structure to which it conforms. In the context of the present application, optical fiber 420 is typically used to assess a three-dimensional shape of the greater palatine canal and monitor advancement and navigation of implant 320 distally in the canal by monitoring changes in the shape of optical fiber 420 during advancement.

Additionally or alternatively, optical fiber 420 is used to assess a position of neural stimulator implant 320 within the greater palatine canal based on a shape of the optical fiber during advancement. Typically, use of optical fiber 420 facilitates verifying orientation and location of implant 320 during and following deployment of the implant in the vicinity of the SPG, such that a post-operative CT scan is in many cases not necessary.

As noted hereinabove, optical fiber 420 is optically couplable to optical fiber shape-sensing system 426. The optical fiber shape-sensing system typically comprises fiber shape-sensing circuitry configured to process the optical signal from the optical fiber and generate an output indicative of the dynamic shape of optical fiber 420. (Typically the optical fiber shape-sensing circuitry 428 comprises a circuitry unit intended for multiple uses.) It will be appreciated that circuitry 428 may be standard circuitry of a multi-purpose computer, which performs the desired shape sensing operations due to software running on the computer.

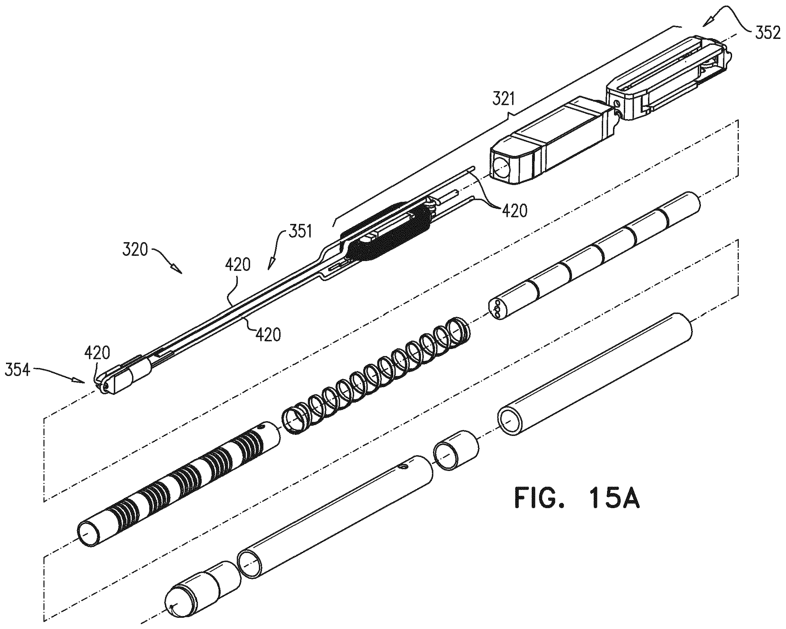

Fig. 15A is a schematic illustration of implant 320 and optical fiber 420. Typically optical fiber 420 contacts implant 320. For example, optical fiber 420 may be wrapped around a portion of neural stimulator implant 320, e.g., wrapped around distal portion 354 of the implant.

For some applications, optical fiber 420 is fixed to neural stimulator implant 320, and can only be separated from the implant by permanently changing a component (e.g., by cutting the fiber). In these applications, at least a portion of optical fiber 420 remains implanted at the SPG. For some applications, optical fiber 420 is shaped to define a predetermined breaking point 490 of optical fiber 420 between a distal portion 494 of optical fiber 420 and a proximal portion 492 of optical fiber 420, such that application of force to predetermined breaking point 490 causes breaking of optical fiber 420 at predetermined breaking point 490. For some applications, predetermined breaking point 490 is shaped to define a narrow portion of optical fiber 420. Breaking of optical fiber 420 at predetermined breaking point 490 typically facilitates separating of proximal portion 492 from distal portion 494 (e.g., by pulling), and subsequent removal of proximal portion 492 from the palatine canal. Distal portion 494 typically remains implanted at the SPG (typically along with neural stimulator implant 320).

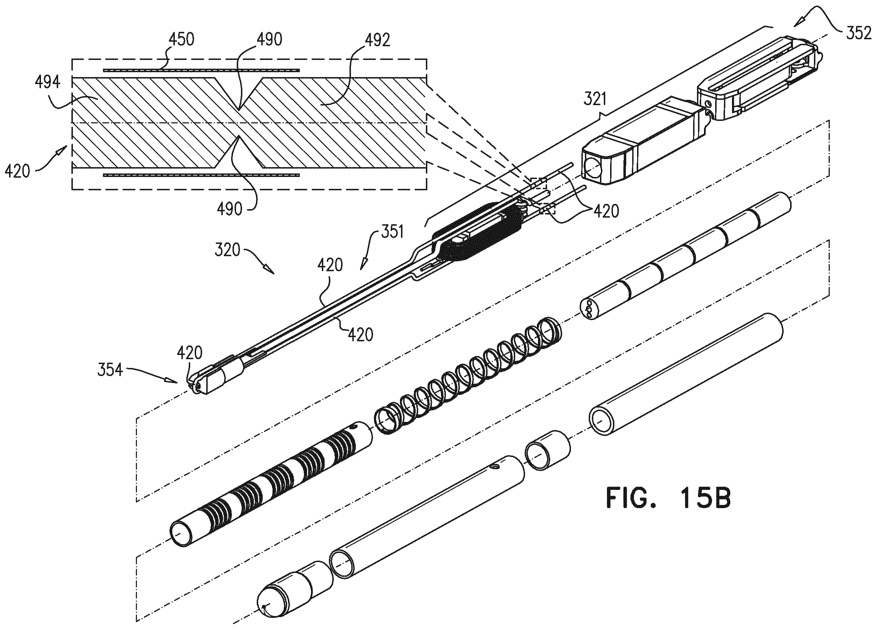

For some applications, optical fiber 420 is shaped to define more than one predetermined breaking point 490. For example, when optical fiber 420 is wrapped around a portion of neural stimulator implant 320, as shown in Figs. 15A and 15B, optical fiber 420 may have a second predetermined breaking point 490 allowing removal of both proximal ends of optical fiber 420.

For some applications, optical fiber 420 is partly disposed within a sheath 450 such that predetermined breaking point 490 is disposed within sheath 450. Typically, some or all residue that may occur as a result of breaking of fiber 420 at predetermined breaking point 490 is contained in sheath 450.

Typically sheath 450 has a length of at least 2 mm and/or less than 15 mm, e.g., at least 5 mm and/or less than 10 mm. For some applications, as shown in Fig. 15B, only a small portion of proximal portion 492 is disposed in sheath 450, facilitating ease of removal of proximal portion 492 from the palatine canal.

Typically, sheath 450 stays in place after breaking of fiber 420 and removal of proximal portion 492 from the palatine canal. For some applications, sheath 450 is mechanically coupled to distal portion 494, e.g., glued to distal portion 494 or held by friction to distal portion 494, thus keeping sheath 450 in place after breaking of fiber 420.

Alternatively, sheath 450 may be pulled out of the palatine canal along with proximal portion 492. For example, the position of predetermined breaking point 490 within sheath 450 may define whether sheath 450 slides out of the palatine canal along with proximal portion 492 when proximal portion 492 is pulled proximally. For example, if most of the portion of fiber 420 that is disposed in sheath 450 is part of proximal portion 492, sheath 450 is likely to be pulled out of the palatine canal along with proximal portion 492.

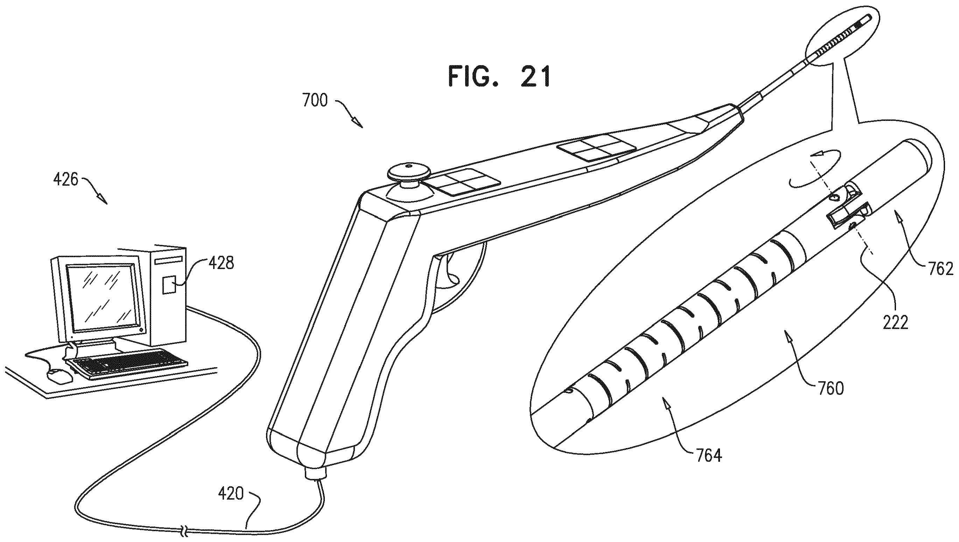

Alternatively or additionally, for some applications, optical fiber 420 is coupled to tool 700 and is not in contact with implant 320, and is advanced distally in the greater palatine canal while coupled to tool 700. Applications in which optical fiber 420 is coupled to tool 700 and is advanced distally in the greater palatine canal in the absence of implant 320 are described hereinbelow with reference to Fig. 21.

Figs. 15A and 15B show proximal, distal and middle portions of implant 320, in accordance with some applications of the present invention. Typically proximal portion 352 is more rigid than middle portion 351. Additionally, distal portion 354 is more rigid than middle portion 351 (middle portion 351 typically includes connecting element 328 described hereinabove with reference to Fig. 10).

Reference is again made to Figs. 14A-B. Tool 700 is typically removably coupled to implant 320 and is configured to deliver the implant to the SPG through the greater palatine canal. Following deployment of implant 320 in the vicinity of the SPG, tool 700 detaches from implant 320. For some applications, tool 700 comprises a detachment mechanism configured to detach the delivery tool from implant 320 (e.g., a spring-based release mechanism, as is generally known in the art). For some applications, the cutting tool cuts fiber 420 without detaching implant 320 from tool 700, and typically after implant 320 has been detached from tool 700. For some applications, the detachment mechanism comprises a cutting tool, configured to detach tool 700 from the implant by cutting optical fiber 420.

For some applications, in which optical fiber 420 is not fixed to implant 320, optical fiber 420 is decoupled from the neural stimulator implant by pulling a proximal end of the optical fiber. Typically, while pulling optical fiber 420, implant 320 is maintained in place by tool 700 (or by additional mechanical elements). For example, a pusher may be used to maintain implant 320 in the vicinity of the SPG, while optical fiber 420 is being pulled and decoupled from implant 320. As shown for example in Fig. 15, optical fiber 420 wraps around the distal end of implant 320, such that by pulling optical fiber 420 while holding implant 320 in place, optical fiber 420 is entirely removed from contact with the implant.

It is noted that the scope of the present invention includes using optical fiber 420, even without an implant, to assess a shape of a bony canal (not necessarily the greater palatine canal), by assessing a shape of the optical fiber during advancement through the bony canal.

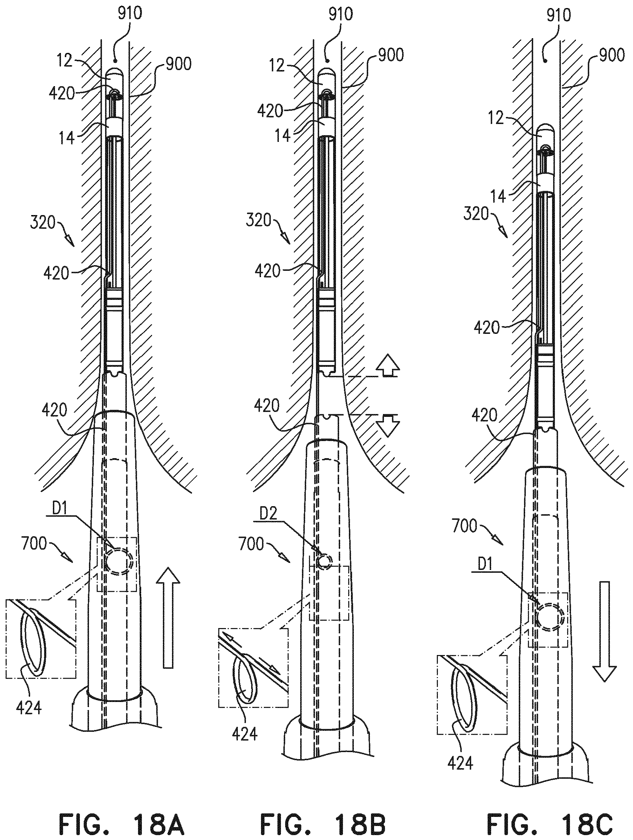

Reference is now made to Figs. 18A-C, which are schematic illustrations of apparatus 200 comprising neural stimulator implant 320, optical fiber 420 (removably coupled or fixed to implant 320) and tool 700 in accordance with some applications of the present invention. As shown in Figs. 18A-C, optical fiber 420 is used to assess proper detachment of implant 320 from tool 700. Typically, once delivery tool 700 reaches the implantation site (i.e., the vicinity of the SPG), implant 320 is deployed at the implantation site by detaching from tool 700. Tool 700 is subsequently pulled back through the greater palatine canal and removed from the body of the subject. In cases in which detachment of implant 320 from tool 700 is not complete, implant 320 may be (undesirably) pulled back proximally in the canal together with tool 700, instead of properly remaining at the implantation site. Thus, for some applications, optical fiber 420 is used to assess proper detachment of implant 320 from tool 700 by monitoring a change in a shape of fiber 420. For such applications, a portion of optical fiber 420 is disposed in tool 700 and is shaped within tool 700 such that relative motion between implant 320 and delivery tool 700 (e.g., distancing of tool 700 from implant 320) causes a change in the shape of the portion of the optical fiber in tool 700.

As shown in Figs. 18A-C, for some applications, the portion of optical fiber 420 in tool 700 is shaped to define a loop 424, such that distancing of the delivery tool from the implant causes a reduction in a diameter of the loop. Fig. 18A shows implant 320 coupled to optical fiber 420 and mounted onto tool 700 while being distally advanced in greater palatine canal 900. A portion of fiber 420 is additionally disposed in tool 700 and is shaped to define a loop 424 during distal advancement of tool 700. When proper detachment of implant 320 from tool 700 is achieved at implantation site 910, tool 700 is distanced from implant 320 by slightly pulling tool 700 back in the canal. Distancing of the tool from the implant naturally causes a reduction in a diameter of loop 424, e.g., from D1 to D2 (Fig. 18B), since fiber 420 is held on either end by tool 700 and implant 320 while they are separating. The reduction in the diameter of loop 424 typically indicates proper decoupling of tool 700 from implant 320. Once proper decoupling is indicated, fiber 420 is cut at the distal portion of tool 700, and tool 700 is removed from the body of the subject. When proper decoupling of tool 700 and implant 320 is not achieved (Fig. 18C), this is indicated by tool 700 being pulled proximally in the canal together with implant 320, and a diameter of loop 424 remaining generally unchanged, indicating insufficient detachment of implant 320 from tool 700.

It is noted that loop 424 is shown by way of illustration and not limitation. The scope of the present invention includes additional or alternative non-straight shapes of the portion of fiber 420 that is disposed in tool 700. For example, the portion of optical fiber 420 in delivery tool 700 may be shaped to define a curve, such that distancing of delivery tool 700 from implant 320 causes a straightening of the curve.

Additionally or alternatively, apparatus 200 further comprises a proximity sensor configured to indicate that tool 700 is detached from implant 320 by generating a signal that varies in response to relative motion (e.g., distancing) between delivery tool 700 and implant 320.

For some applications, the proximity sensor comprises at least one radiofrequency (RF) coil coupled to implant 320, and at least one RF coil coupled to delivery tool 700. For example, the RF coil coupled to tool 700 may transmit energy to the RF coil which is coupled to implant 320. Typically, the RF receiving coil which is coupled to the implant has a load modulation circuit which imposes changes in the transmission signal, which are detected by the transmitting coil coupled to tool 700. Thus, the RF coil coupled to tool 700, e.g., at a distal end of tool 700, receives a baseline level of feedback from the RF coil coupled to implant 320 when implant 320 is mounted onto tool 700. A difference in the feedback from the RF coil coupled to the implant and received by tool 700 typically indicates proper separation of implant 320 from tool 700. On the other hand, pulling back of tool 700 without a change (or without a sufficient change) in the feedback typically indicates insufficient detachment between implant 320 and tool 700.

For some applications, the proximity sensor comprises at least one magnetic element coupled to implant 320 and at least one magnetic element coupled to the delivery tool 700. A sufficient change in the magnetic force between the two elements indicates sufficient detachment.

It is noted that the proximity sensor can be used in combination with, or in the absence of, optical fiber 420. The proximity sensor is used to indicate that delivery tool 700 is detached from the implant by generating a signal in response to relative motion (e.g., distancing) between delivery tool 700 and implant 320. For such applications, and in general, implant 320 typically has a maximum length of 4 cm and/or a maximum diameter of 3 mm.

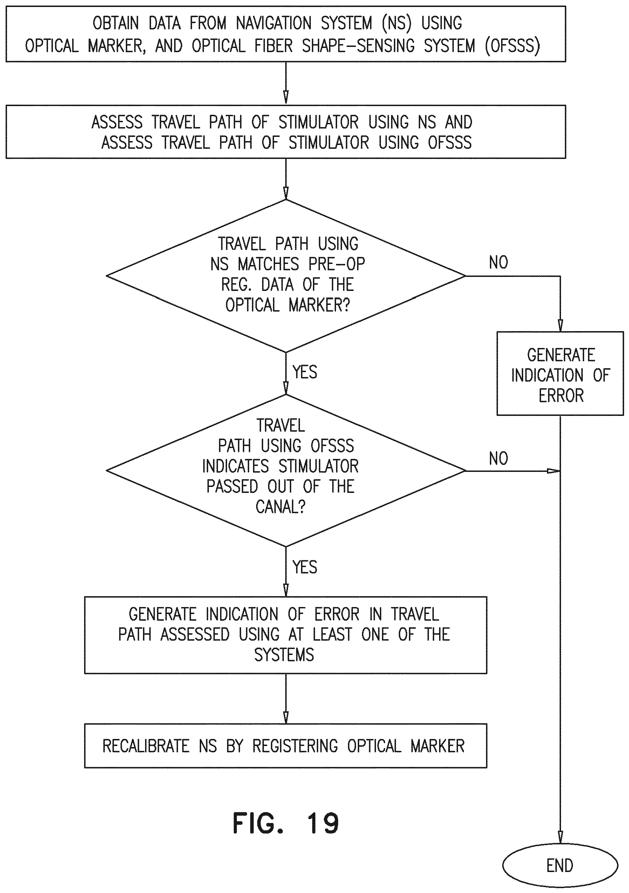

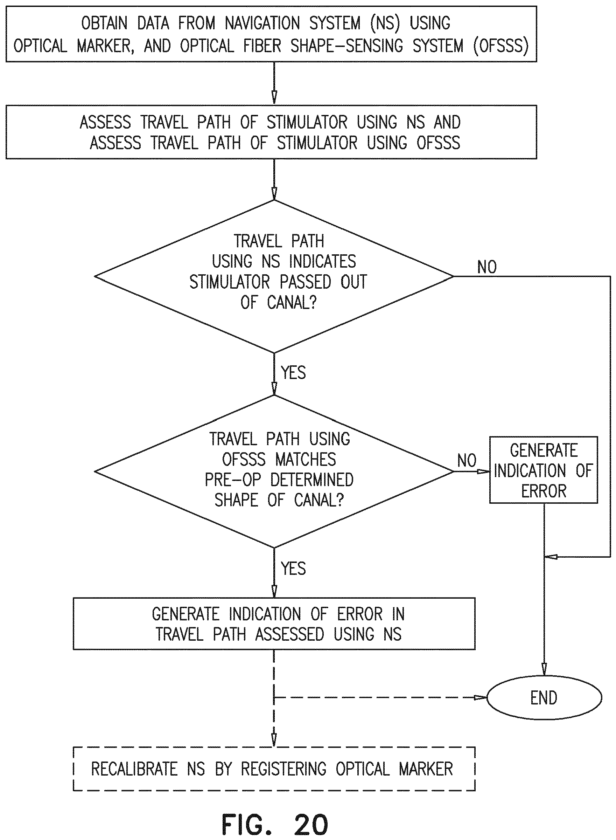

Reference is made to Figs. 19 and 20, which are flow charts of steps for a method provided, in accordance with some applications of the present invention. For some applications, a travel path of implant 320 through the greater palatine canal is assessed by navigation system (NS), e.g., using optical markers 500, 510 and 520 on tool 700. Additionally, a travel path of implant 320 is assessed by the optical fiber shape sensing system (OFSSS) based on shape changes of the optical fiber during advancement to the SPG, as described herein. Typically it is possible to indicate whether there is an error in the travel path assessed using the navigation system if (a) the travel path detected using the optical fiber shape-sensing system matches a preoperatively determined shape of the canal, typically obtained by CT-scan, and (b) the travel path assessed using the navigation system indicates that the neural stimulator has passed out of the canal. Fig. 20 is a flow chart showing the above described steps for indicating whether there is an error in the travel path assessed using the navigation system (NS), in accordance with some applications of the present invention.

Alternatively or additionally, the scope of the present invention includes indicating whether there is an error in the travel path assessed using at least one of the systems (i.e., NS and OFSSS) if (a) the travel path assessed using the navigation system matches pre-operative registration data of the optical marker, and (b) the travel path detected using the optical fiber shape-sensing system indicates that the neural stimulator has passed out of the canal. In cases in which an error is indicated, the navigation system is typically recalibrated by performing registration of the optical markers. Fig. 19 is a flow chart showing steps for indicating whether there is an error in the travel path assessed using at least one of the systems (i.e., NS and OFSSS).

Reference is now made to Fig 21. For some applications, optical fiber 420 is distally advanced in the palatine canal without neural stimulator implant 320. For some such applications, delivery tool 700 comprises a steerable delivery device 760, e.g., a steerable trocar. As shown in Fig. 21, steerable delivery device 760 comprises axis 222 at a hinge of steerable delivery device 760, allowing steering of distal portion 762 of device 760. Portion 764 of steerable delivery device 760 is typically flexible, facilitating advancement through the palatine canal based on the steering of distal portion 762. Optical fiber 420 is typically distally advanced through the palatine canal using steerable delivery device 760 of tool 700.

A shape of the canal is assessed, e.g., mapped, using optical fiber shape-sensing system 426, based on a shape of optical fiber 420 during advancement through the palatine canal. Typically, steerable delivery device 760 is navigated in the palatine canal based on the assessing of the shape of the palatine canal. Accordingly, use of optical fiber 420 typically facilitates safe steering of delivery device 760 in the palatine canal, avoiding injuring to the palatine canal. Steerable delivery device 760 may be steered by deflection of a distal end of steerable delivery device 760.

Optical fiber 420 is then removed from the palatine canal and subsequently to removal of fiber 420, neural stimulator implant 320 is distally advanced using delivery tool 700, with or without delivery device 760, or using another delivery tool, through the palatine canal, and is implanted at a vicinity of the sphenopalatine ganglion (SPG) to apply electrical stimulation thereto. Typically, neural stimulator implant 320 is navigated through the palatine canal based on assessing of the shape of the palatine canal with optical fiber 420.

As described hereinabove, for some applications, a proximity sensor is configured to indicate that delivery tool 700 is detached from implant 320 by generating a signal that varies in response to relative motion (e.g., distancing) between delivery tool 700 and implant 320.

For some applications, prior to advancement of neural stimulator implant 320, delivery device 760 is advanced in the palatine canal together with fiber 420 and is used to prepare the palatine canal for subsequent advancement of neural stimulator implant 320, e.g., by widening the palatine canal. It is noted that preparation of the palatine canal with delivery device 760 and fiber 420 in the absence of neural stimulator implant 320 avoids forces of tissue clearance during preparation of the canal from being applied to neural stimulator implant 320. It is noted however, that for other applications as described for example in Figs. 15A-B, neural stimulator implant 320 is advanced with fiber 420 and the delivery tool.

For some applications, for example, when a shape of the palatine canal is assessed by optical fiber 420 and/or by pre-operative CT as having many curves that would make advancing of neural stimulator implant 320 therethrough difficult, neural stimulator implant 320 may be advanced out of the palatine canal through a naturally-occurring or a surgically-made hole in the canal. In such cases, neural stimulator implant 320 is advanced out of the palatine canal, and for example, parallel to the canal, through the maxillary sinus which is located lateral to the palatine canal, toward the sphenopalatine ganglion (SPG).

Reference is again made to Figs 14-21. It is noted that shape sensing of the palatine canal with fiber 420 as described herein is performed on a sensing length of 4-6 cm (e.g., 5 cm) of fiber 420, which is the approximate length of the palatine canal. Thus, for example, the fiber Bragg gratings for use for these applications are typically positioned 2-5 mm apart.

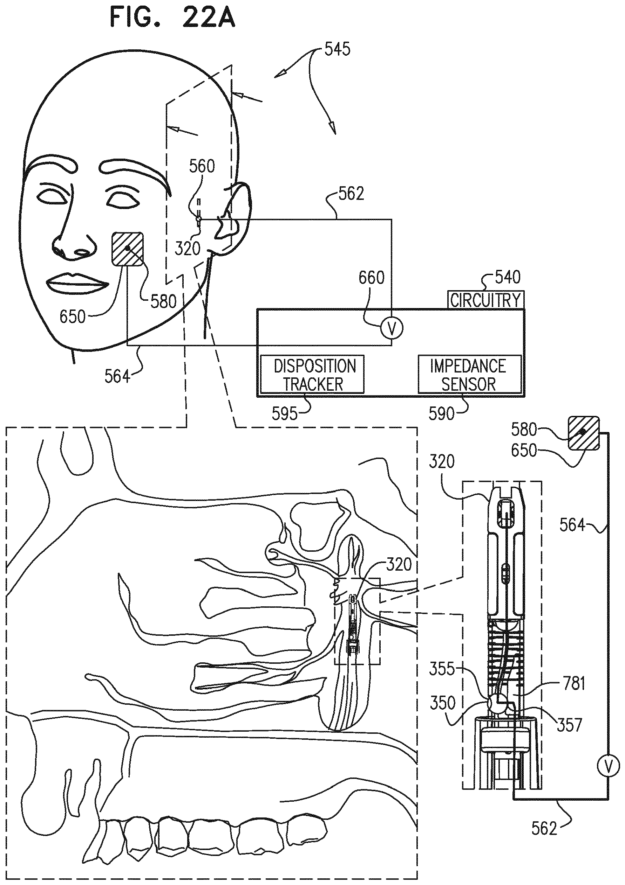

Reference is now made to Figs. 22A-B, which are schematic illustrations of apparatus comprising an impedance-based navigation system 545. Figs. 22A-B show neural stimulator implant 320 in the greater palatine canal and connected to impedance-based navigation system 545. For some applications, navigation of neural stimulator implant 320 to the SPG through the greater palatine canal is facilitated by impedance-based navigation system 545. Impedance-based navigation system 545 typically comprises impedance-based navigation circuitry 540 which assesses a disposition, e.g., a location, a shape and/or an orientation, of neural stimulator implant 320 in the greater palatine canal (or outside of the greater palatine canal). For some applications, impedance-based navigation is used in combination with navigation techniques described herein with reference to optical-marker-based navigation and/or optical fiber shape sensing-based navigation.

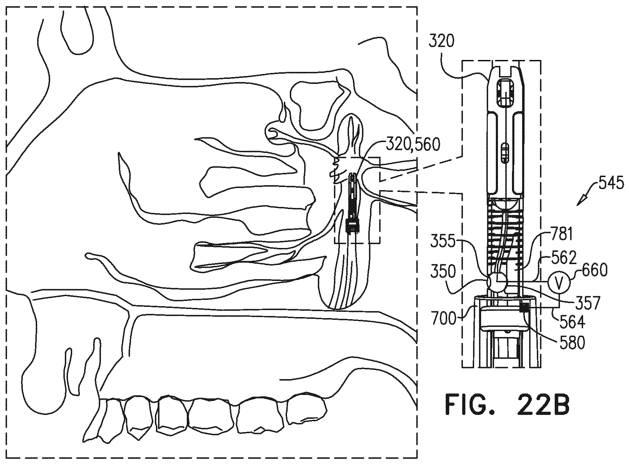

For such applications, implantable neural stimulator 320 has an implant-impedance-sensing electrode 560, and impendence is measured between implant-impedance-sensing electrode 560 and an auxiliary-impedance-sensing electrode 580. For some applications, auxiliary-impedance-sensing electrode 580 is disposed on an adhesive patch 650 positioned on a face of the subject (Fig. 22A). For other applications, auxiliary-impedance-sensing electrode 580 is coupled to delivery tool 700 (Fig. 22B).

Impedance-based navigation circuitry 540 typically comprises a voltage generator 660 which applies current between implant-impedance-sensing electrode 560 and auxiliary-impedance-sensing electrode 580 through first and second wires 562 and 564 which are electrically coupled respectively to implant-impedance-sensing electrode 560 and auxiliary-impedance-sensing electrode 580. (For clarity of illustration, in one of the views, wire 562 is schematically shown going through the patient's skin, whereas wire 562 is typically passed along with the implant through greater palatine canal 900.) Impedance-based navigation circuitry 540 typically further comprises an impedance sensor 590 configured to measure impedance between implant-impedance-sensing 560 and auxiliary-impedance-sensing electrodes and 580, based on the applying of the current from voltage generator 660. Impedance-based navigation circuitry 540 further comprises a disposition tracker 595 configured to determine, based on the impedance measurements, e.g., based on a change in the measured impedance, a disposition of neural stimulator implant 320 in the greater palatine canal.

For some applications, implant-impedance-sensing electrode 560 is configured to stimulate the SPG. For such applications, neural stimulator implant 320 comprises stimulation circuitry configured to drive implant-impedance-sensing electrode 560 to apply electrical stimulation to the sphenopalatine ganglion (SPG) of the subject. Typically, impedance-based navigation system 545 is decoupled from neural stimulator implant 320 (typically by decoupling first wire 562 from implant-impedance-sensing electrode 560), once neural stimulator implant 320 is delivered to the SPG. Following decoupling of navigation system 545, the stimulation circuitry in neural stimulator implant 320 typically drives implant-impedance-sensing electrode 560 to apply electrical stimulation to the SPG.

Additionally or alternatively, implantable neural stimulator 320 comprises at least two stimulating electrodes configured to stimulate the SPG (e.g., electrodes 12 and 14 described herein with reference to Figs. 9A-B), and implant-impedance-sensing electrode 560 does not function as a stimulating electrode. For such applications, stimulation circuitry drives stimulating electrodes 12 and 14 to apply electrical stimulation to the sphenopalatine ganglion, but implantable neural stimulator 320 does not comprise circuitry to drive implant-impedance-sensing electrode 560 to apply electrical stimulation to the sphenopalatine ganglion. Optionally, impedance-based navigation circuitry 540 utilizes impedance measurements with respect to implant-impedance-sensing electrode 560 and not with respect to any other electrode of the implantable neural stimulator (e.g., electrodes 12 and 14).

Fig. 22A shows auxiliary-impedance-sensing electrode 580 coupled to adhesive patch 650 and placed on the face of the subject, by way of illustration and not limitation. For some applications, voltage generator 660 and navigation circuitry 540 is coupled to a proximal portion of stimulator 320 and auxiliary-impedance-sensing electrode 580 is coupled to delivery tool 700, and is distally advanced through the greater palatine canal together with delivery tool 700 (as shown in Fig. 22B). Impendence is measured between implant-impedance-sensing electrode 560 and auxiliary-impedance-sensing electrode 580, and a disposition of neural stimulator 320 is assessed based on the impedance measurements.

As described hereinabove, the disposition of implantable neural stimulator 320 may include an orientation of implantable neural stimulator 320 in the greater palatine canal, and disposition tracker 595 determines the orientation of the implantable neural stimulator in the greater palatine canal based on the impedance measurements, e.g., based on a change in the measured impedance.

Additionally or alternatively, the disposition of implantable neural stimulator 320 includes a location of implantable neural stimulator 320 in the greater palatine canal, and disposition tracker 595 determines the location of implantable neural stimulator 320 in the greater palatine canal based on based on the impedance measurements, e.g., based on a change in the measured impedance.

Further additionally or alternatively, the disposition of implantable neural stimulator 320 includes a shape of the implantable neural stimulator in the greater palatine canal, and disposition tracker 595 is configured to determine the shape of implantable neural stimulator 320 in the greater palatine canal based on a change in the measured impedance. For some applications, disposition tracker 595 determines the shape of the implantable neural stimulator by tracking successive locations of implantable neural stimulator 320 in the greater palatine canal.

For some applications, impedance-based navigation circuitry 540 is configured to generate an output if (i) a distal portion of implantable neural stimulator 320 is not advancing in the greater palatine canal and (ii) a proximal portion of the neural stimulator is advancing in the canal, and disposition tracker 595 determines the shape of implantable neural stimulator 320 based on the output generated by impedance-based navigation circuitry 540.

For some applications, implantable neural stimulator 320 has a proximal portion, a distal portion and a middle portion between the proximal and distal portions, the proximal and distal portions being more rigid than the middle portion. Typically, implant-impedance-sensing electrode 560 is a first implant-impedance-sensing electrode 560 and is disposed on the distal portion of implantable neural stimulator 320. Additionally, neural stimulator 320 further comprises a second implant-impedance-sensing electrode 560, disposed on the proximal portion of neural stimulator 320. Voltage generator 660 applies current between auxiliary-impedance-sensing 580 and (in alternation) first and second implant-impedance-sensing electrodes 560. Impedance sensor 590 measures respective impedances between auxiliary-impedance-sensing 580 and first and second implant-impedance-sensing electrodes 560 based on the applying of the current by voltage generator 660. Disposition tracker 595 then determines the shape of implantable neural stimulator 320 in the greater palatine canal, based on: (a) a change in the impedance measured between first implant-impedance-sensing electrode 560 and auxiliary-impedance-sensing electrode 580, and (b) a change in the impedance measured between second implant-impedance-sensing 560 and auxiliary-impedance-sensing electrodes 580.

For some applications, both implant-impedance-sensing electrode 560 and auxiliary-impedance-sensing electrode 580 are disposed on implantable neural stimulator 320. For example, neural stimulator 320 may have a proximal portion, a distal portion and a middle portion between the proximal and distal portions, the proximal and distal portions being more rigid than the middle portion. Implant-impedance-sensing electrode 560 is typically disposed on the distal portion of neural stimulator 320, and auxiliary-impedance-sensing electrode 580 is disposed on the proximal portion of neural stimulator 320. For such applications, impedance sensor 590 measures an impedance between auxiliary-impedance-sensing 580 and implant-impedance-sensing electrode 560 based on the applying of the current by voltage generator 660. Disposition tracker 595 then determines the shape of implantable neural stimulator 320 in the greater palatine canal, based on a change in the impedance measured between implant-impedance-sensing electrode 560 and auxiliary-impedance-sensing electrode 580.

Reference is again made to locking element 350, which is shown in Figs. 22A-B and is described hereinabove. For some applications, locking element 350 (also shown in Fig. 8) electrically couples first wire 562 to implant-impedance-sensing electrode 560, and a locking element controller, e.g., engaging element 781 (also shown in Fig. 8), is configured to disengage locking element 350 from first wire 562. Disengaging locking element 350 from first wire 562 allows withdrawing wire 562 from the greater palatine canal, generally without dislodging implant 320 from its implantation location near the SPG.

As shown in Figs. 8 and 22A-B, for some applications, locking element 350 is shaped as a ball. Typically, locking element 350 is in electrical contact (and typically also in physical contact) with first wire 562, but is not fixed to first wire 562, thereby allowing first wire 562 to be withdrawn from the greater palatine canal, generally without dislodging implant 320 from its implantation location near the SPG.

Typically, locking element 350 is not in electrical contact with any component of the apparatus except via first wire 562 or via implantable neural stimulator 320. Locking element 350 typically has an outer surface having first and second portions 357 and 355 respectively. First portion 357 is typically non-insulated and in electrical contact with first wire 562, and second portion 355 is insulated. Locking element 350 is typically insulated such that when implantable neural 320 is in the greater palatine canal, locking element 350 is not in direct electrical contact with any portion of anatomy of the subject.

Reference is still made to Figs. 22A-B. It is noted that for some applications, more than one implant-impedance-sensing electrode 560, e.g., at least one second implant-impedance-sensing electrode, is coupled to implantable neural stimulator 320. For example, a plurality of second implant-impedance-sensing electrodes may be coupled to implantable neural stimulator 320. Typically, impedance sensor 590 is configured to measure impedance between the at least one second implant-impedance-sensing electrode and another electrode (e.g., auxiliary-impedance-sensing electrode 580, disposed on a portion of the face of the subject, or on delivery tool 700 as described herein). It is generally noted that there may be more than one, e.g., more than two, implant-impedance-sensing electrodes coupled to implantable neural stimulator 320, and there may be more than one auxiliary-impedance-sensing electrode 580. For example, there may be additional auxiliary-impedance-sensing electrodes 580 disposed on adhesive patches placed on several locations on the face. Similarly, there may be additional auxiliary-impedance-sensing electrodes 580 disposed on delivery tool 700.

For some applications, implantable neural stimulator 320 comprises stimulation circuitry configured to drive the first and second implant-impedance-sensing electrodes to apply electrical stimulation to the sphenopalatine ganglion (SPG) of the subject. For other applications, implantable neural stimulator 320 does not comprise circuitry to drive the second implant-impedance-sensing electrode to apply electrical stimulation to the sphenopalatine ganglion (SPG) of the subject.

For some applications, the second implant-impedance-sensing electrode is coupled to voltage generator 660 through first wire 562, and a multiplexer (not shown) coupled to the stimulator selectively applies current from first wire 562 to the first and second implant-impedance-sensing electrodes.

Reference is now made to Figs. 22A-B and Figs. 2-3H. For some applications, impedance-based navigation system 545 is used in combination with oral surgical guide 40. As described hereinabove oral surgical guide 40 guides stimulator 320 through the greater palatine foramen of the palate of the oral cavity and into the greater palatine canal at an angle that is suitable for entering the greater palatine canal. Disposition tracker 595 typically receives as an input an indication of the angle, and determines the disposition of implantable neural stimulator 320 in the greater palatine canal based on the indication of the angle, i.e., based on knowing the angle at which implantable neural stimulator 320 entered the canal.

Reference is still made to Figs. 22A-B. It is noted that although impedance-based navigation circuitry 540 is described as calculating impedance based on measuring current (e.g., measuring the amplitude and the phase of current) between two electrodes (e.g., implant-impedance-sensing electrode 560 and auxiliary-impedance-sensing electrode 580), it is understood that any other way of calculating impedance may be used to achieve impedance measurements for facilitating navigation of neural stimulator 320.

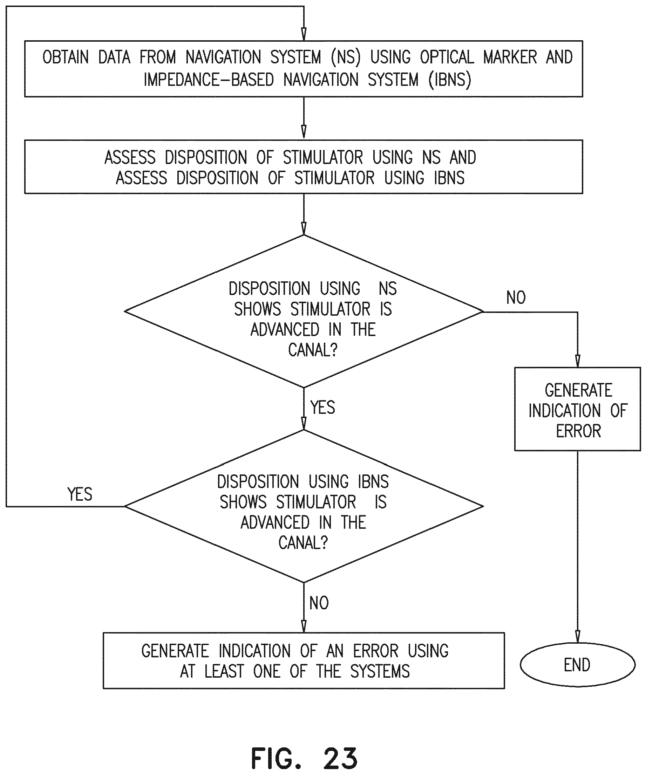

Reference is now made to Fig. 23. For some applications, impedance-based navigation system 545 is used in combination with an optical-based navigation system e.g., using optical markers 500, 510 and 520 on delivery tool 700 (or an encoder), as described hereinabove. The optical markers typically track movement of neural stimulator 320 through the greater palatine canal. Disposition tracker 595 of impedance-based navigation circuitry 540 receives information from the optical-based navigation system and determines based on the information from the optical-based navigation system and based on the measured impedance by circuitry 540 whether implantable neural stimulator 320 is stuck in the greater palatine canal.

For example, the optical-based navigation system, may assess a disposition of neural stimulator 320 in the canal based on movement of the optical marker attached to delivery tool 700, while the tool is advanced distally in the canal. In addition, a disposition of neural stimulator 320 is assessed by disposition tracker 595 using impedance measurements as described herein. Typically it is possible to indicate whether there is an error in the disposition of neural stimulator 320 assessed using the optical-based navigation system if (a) no change in impedance measurements is assessed by impedance-based navigation circuitry 540 and (b) the disposition assessed using the optical-based navigation system indicates that neural stimulator 320 is being advanced in the canal.

For example, generating an indication that there is an error in the disposition assessed using the optical-based navigation system may comprise generating an indication that the implantable neural stimulator is stuck in the canal, e.g., is being pushed against a wall of the canal such that it cannot be advanced distally in the canal to the SPG. In such a case, the optical-based navigation system shows movement of neural stimulator 320, but no change in impedance measurements is assessed by impedance-based navigation circuitry 540. For example, neural stimulator 320 may get stuck in the greater palatine canal when reaching a curved area in the canal. In such cases, implantable neural stimulator 320 may get stuck against a wall of the canal. A shape of neural stimulator 320 is typically deformed in response to being pushed against a wall of the canal.

As shown in the flow chart in Fig. 23, a disposition of implant 320 in the greater palatine canal is assessed by a navigation system (NS), e.g., using optical markers 500, 510 and 520 on tool 700 using techniques and apparatus described hereinabove. Additionally, a disposition of implant 320 is assessed by the impedance-based navigation system (IBNS) based on measured impedance during advancement of the stimulator to the SPG, as described herein. As described, it is possible to indicate whether there is an error in the disposition assessed using the navigation system if (a) no change in impedance measurements is assessed by the impedance-based navigation system and (b) the disposition assessed using the optical-based navigation system indicates that neural stimulator 320 is being advanced in the canal.

Additionally or alternatively, impedance-based navigation circuitry 540 uses the impedance measurements to assess whether implantable neural stimulator 320 has passed out of the canal based on a change in the measured impedance. Neural stimulator implant 320 may be advanced out of the palatine canal through a naturally-occurring or a procedurally-created hole in the canal. In such cases, neural stimulator implant 320 may be unintentionally advanced out of the palatine canal, for example, into the maxillary sinus which is located lateral to the palatine canal. Typically, a sharp change (e.g., a sudden rise) in impedance measurements indicates that neural stimulator implant 320 has advanced out of the palatine canal. Similarly, neural stimulator implant 320 may be advanced out of the palatine canal into the nasal cavity, or nasopharynx, and this typically-undesired occurrence is detected, using the techniques described, based on a rise in the measured impedance. For example, for some applications, a first and second implant-impedance-sensing electrodes 560 are both disposed on the distal portion of implantable neural stimulator 320, and impedance-based navigation circuitry 540 determines whether neural stimulator 320 has passed out of the greater palatine canal based on a change in the impedance measured between the first implant-impedance-sensing and second-impedance-sensing electrodes.

Additionally or alternatively to determining whether neural stimulator 320 has passed out of the greater palatine canal, it is noted that impedance measurements between the first and second implant-impedance-sensing electrodes 560 both disposed on the distal portion of stimulator 320, may be used by impedance-based navigation circuitry 540 to facilitate navigation of neural stimulator 320 in the greater palatine canal.

For some applications, CT scan data of the subject is used in combination with impedance-based navigation system 545 to assess a disposition of neural stimulator 320 in the greater palatine canal. For such applications, navigation of implantable neural stimulator 320 in the greater palatine canal is based on the CT scan data of the subject indicating a shape of the greater palatine canal (e.g., indicating curves in the canal), and on an output from impedance-based navigation circuitry 540. Typically, implantable neural stimulator 320 is configured to curve in the greater palatine canal in accordance with a curvature of the greater palatine canal as indicated by the CT scan data. Typically, impedance-based navigation circuitry 540 generates an indication that implantable neural stimulator 320 has passed out of the greater palatine canal if (i) based on the impedance-based navigation circuitry 540 the shape of the stimulator is straight, and (ii) based on the CT scan data a shape of the greater palatine canal at a present location of the implantable neural stimulator is curved.

It is noted that for applications in which it is indicated that neural stimulator 320 has passed out of the greater palatine canal, the stimulator is typically advanced from its location outside of the canal toward the sphenopalatine ganglion (SPG). In other cases, the stimulator is retracted from the subject's body and reintroduced through the greater palatine foramen into the greater palatine canal.

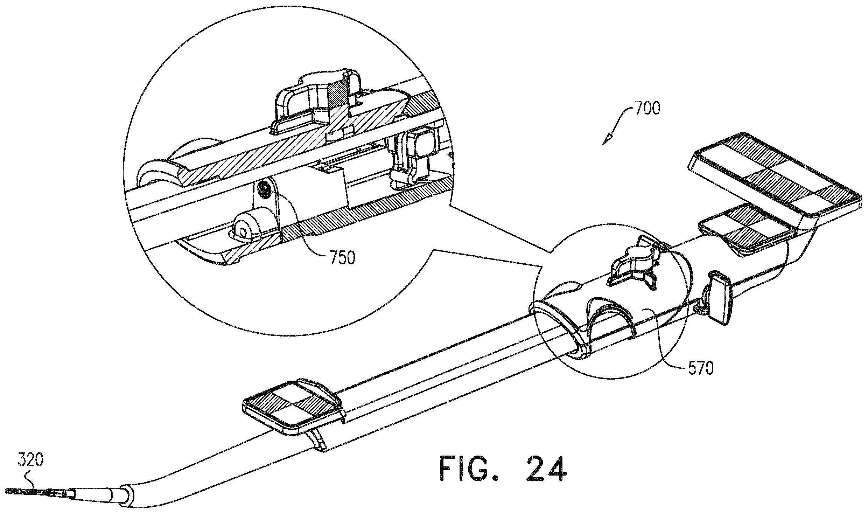

Reference is now made to Fig. 24, which is a schematic illustration of delivery tool 700 comprising an electrical force sensor 750 for facilitating navigation of neural stimulator 320 through the greater palatine canal. For some applications, force sensor 750 measures the force applied to implant 320 by tool 700 during advancement of implant 320 in the greater palatine canal toward the sphenopalatine ganglion (SPG). Circuitry coupled to the force sensor typically generates a force sensor signal in response to a change in the force measurements, e.g., in response to exceeding a threshold of measured force. (The threshold level of force may be, for example, 5-40 grams of force, 40-300 grams of force, or for some applications, 300-1000 grams of force.) Typically, implant 320 is coupled to delivery tool 700 such that distal advancement of slide-bar 570 of delivery tool 700 causes distal advancement of the implant. Typically, the physician advances slide-bar 570 by applying a distally-directed force thereto, thereby producing distal advancement of implant 320. If the force applied to slide-bar 570, and in turn to implant 320, in a distal direction exceeds a predetermined threshold, a signal is generated by force sensor 750 to alert the operating physician to an issue relating to the proper advancement of implant 320. For example, force sensor 750 may generate a signal due to a sudden increase in force measurements as a result of advancement of the implant being impeded. In addition, the sensor is configured to generate a signal in response to an increase in force sensed by sensor 750 in cases in which the physician is applying excessive force during advancement of the implant in the canal even without advancement of implant 320 being impeded (thereby providing the physician with useful feedback regarding the force applied to slide-bar 570).

For some applications, force measurements using sensor 750 are used in combination with navigation techniques described herein with reference to optical-marker-based navigation and/or optical fiber shape sensing-based navigation and/or impedance-based navigation.

For example, force sensor 750 may be used in combination with impedance-based navigation system 545, as described hereinabove. For such applications, a disposition of neural stimulator 320 is assessed by disposition tracker 595 using impedance measurements as described herein. In addition, force measurements from force sensor 750 are used to assess proper advancement of neural stimulator 320 in the greater palatine canal. Typically, it is possible to indicate whether neural stimulator 320 is stuck in the canal (e.g., is being pushed against a wall of the canal such that it cannot be advanced distally in the canal to the SPG), if (a) force sensor 750 indicates an increase in force when slide-bar 570 is being distally advanced, and (b) no change in impedance measurements is assessed by impedance-based navigation circuitry 540. In such cases, implantable neural stimulator 320 may be stuck against a wall of the canal while a shape of neural stimulator 320 is being deformed in response to being pushed against a wall of the canal.

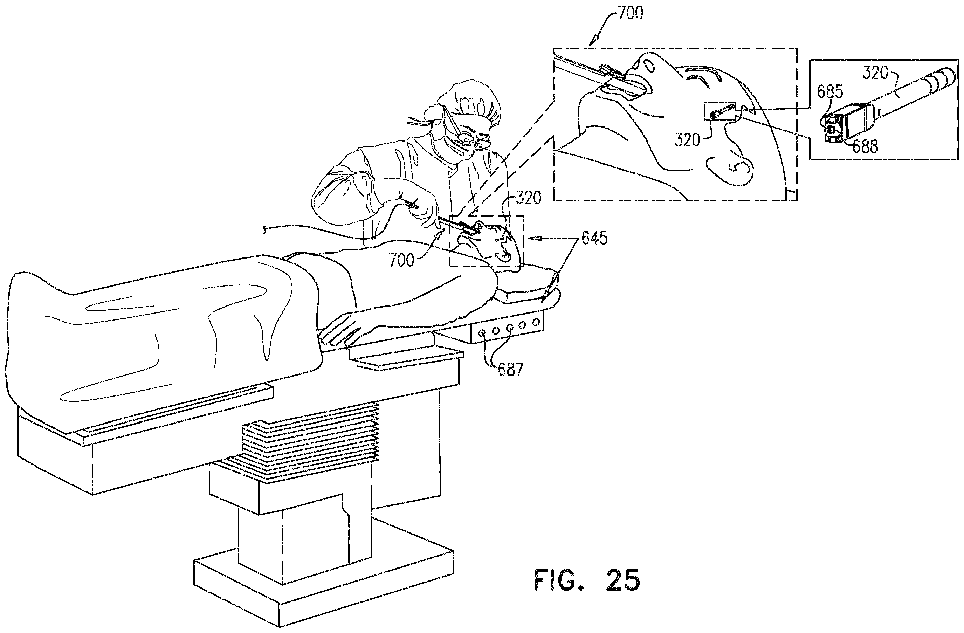

Reference is now made to Fig. 25. For some applications, an electromagnetic-based sensing system 645 is used to determine a disposition, e.g., a location and/or orientation, of implantable neural stimulator 320 in the greater palatine canal. For such applications, at least one sensor coil 685 is coupled to implantable neural stimulator 320, and at least one transmitter coil 687, is disposed outside the subject's body. The at least one transmitter coil 687 generates electromagnetic fields at each of a plurality of field strengths, which induce respective currents in the at least one sensor coil 685. Control circuitry 688 is typically coupled to at least one sensor coil 685, and comprises a current sensor which is coupled to at least one sensor coil 685. The current sensor determines which of the respective induced currents passes a predetermined threshold, and generates a signal in response to determining that the predetermined threshold has been passed. Based on the signal, a disposition tracker determines a disposition, e.g., a location and/or an orientation, of implantable neural stimulator 320 in the greater palatine canal.

Typically, one to six sensor coils 685 are coupled to implantable neural stimulator 320. It is noted however that for some applications, more than six sensor coils 685 are coupled to implantable neural stimulator 320.

Typically, one to six transmitter coils 687 are disposed outside the subject's body. It is noted however that for some applications, more than six transmitter coils 687 are disposed outside the subject's body, and are configured to generate electromagnetic fields at each of a plurality of field strengths, to induce respective currents in the sensor coils on implantable neural stimulator 320.

For some applications, generating of the electromagnetic fields by the transmitter coil 687 is withheld while the control circuitry which is coupled to the sensor coil 685 generates the signal in response to determining that the predetermined threshold has been passed. This typically facilitates minimizing noise and allowing for the small signal generated by the current sensor to be received by the disposition tracker. Typically, the control circuitry comprises an energy storage element, e.g., a battery and/or a capacitor, which stores energy transmitted by transmitter coil 687, and the control circuitry uses the stored energy to power the generation of the signal.

Additionally, or alternatively, electromagnetic-based sensing system 645 is used to determine a disposition, i.e., a location and/or an orientation, of oral surgical guide 40 when it is placed on the dental arch of the subject. Typically, at least one surgical-guide sensor coil 680 (shown in Fig. 3B) is coupled to oral surgical guide 40 and at least one transmitter coil 687, is disposed outside the subject's body. At least one transmitter coil 687 generates electromagnetic fields at each of a plurality of field strengths, which induce respective currents in the at least one surgical-guide sensor coil 680. Surgical-guide circuitry 682 typically comprises a surgical-guide current sensor coupled to the surgical-guide sensor coil and configured to determine whether the current induced in the surgical-guide sensor coil reaches a predetermined threshold. If the predetermined threshold was reached, a surgical-guide circuitry signal is transmitted. A disposition tracker is configured to determine, based on the surgical-guide circuitry signal, a disposition of oral surgical guide 40.

## Claims

1. Apparatus comprising:
an implantable neural stimulator (320) (i) configured to be advanced through a greater palatine canal to a sphenopalatine ganglion (SPG) of a subject and (ii) comprising an implant-impedance-sensing electrode (560);
an auxiliary-impedance-sensing electrode (580);
first and second wires (562, 564), electrically coupled respectively to the implant-impedance-sensing and auxiliary-impedance-sensing electrodes (560, 580); and
impedance-based navigation circuitry (540) comprising:
a voltage generator (660) configured to apply current between the implant-impedance-sensing and auxiliary-impedance-sensing electrodes (560, 580) through the wires (562, 564);
an impedance sensor (590) configured to measure an impedance between the implant-impedance-sensing and auxiliary-impedance-sensing electrodes (560, 580) based on the applying of the current; and
a disposition tracker (595) configured to determine, based on a change in the measured impedance, a disposition of the implantable neural stimulator (320) in the greater palatine canal,
wherein the apparatus is **characterised in that** the disposition of the implantable neural stimulator (320) includes a shape of the implantable neural stimulator (320) in the greater palatine canal, and wherein the disposition tracker (595) is configured to determine the shape of the implantable neural stimulator (320) in the greater palatine canal based on a change in the measured impedance.

2. The apparatus according to claim 1, further comprising:
a locking element (350) electrically coupling the first wire (562) to the implant-impedance-sensing electrode (560); and
a locking element controller configured to disengage the locking element (350) from the first wire (562).

3. The apparatus according to any one of claims 1-2, wherein the first wire (562) is configured to be decoupled from the implant-impedance-sensing electrode (560) once the neural stimulator (320) is delivered to the SPG, and wherein the implantable neural stimulator (320) comprises stimulation circuitry configured to drive the implant-impedance-sensing electrode (560) to apply electrical stimulation to the sphenopalatine ganglion (SPG) of the subject once the neural stimulator (320) is delivered to the SPG, following the decoupling of the first wire (562) from the implant-impedance-sensing electrode (560).

4. The apparatus according to any one of claims 1-2, wherein the implantable neural stimulator (320) further comprises:
at least two stimulating electrodes (12, 14); and
stimulation circuitry configured to drive the at least two stimulating electrodes (12, 14) to apply electrical stimulation to the sphenopalatine ganglion (SPG) of the subject,
wherein the implantable neural stimulator (320) does not comprise circuitry to drive the implant-impedance-sensing electrode (560) to apply electrical stimulation to the sphenopalatine ganglion (SPG) of the subject.

5. The apparatus according to any one of claims 1-2, further comprising a delivery tool (700) configured to distally advance the implantable neural stimulator (320) through the greater palatine canal, wherein the auxiliary-impedance-sensing electrode (580) is coupled to the delivery tool (700).

6. The apparatus according to claim 1, wherein the disposition tracker (595) is configured to determine the shape of the implantable neural stimulator (320) by tracking successive locations of the implantable neural stimulator (320).

7. The apparatus according to claim 1, wherein:
the implantable neural stimulator (320) has a proximal portion, a distal portion and a middle portion between the proximal and distal portions, the proximal and distal portions being more rigid than the middle portion,
the implant-impedance-sensing electrode (560) is disposed on the distal portion of the neural stimulator (320), and
the auxiliary-impedance-sensing electrode (580) is disposed on the proximal portion of the neural stimulator (320).

8. The apparatus according to claim 1, wherein the impedance-based navigation circuitry (540) is configured to:
generate an output if (i) a distal portion of the implantable neural stimulator (320) is not advancing in the greater palatine canal and (ii) a proximal portion of the implantable neural stimulator (320) is advancing in the greater palatine canal, and wherein
the disposition tracker (595) is configured to determine the shape of the implantable neural stimulator (320) based on the output.

9. The apparatus according to any one of claims 1-2, wherein the disposition of the implantable neural stimulator (320) includes a location of the implantable neural stimulator (320) in the greater palatine canal, and wherein the disposition tracker (595) is configured to determine the location of the implantable neural stimulator (320) in the greater palatine canal based on a change in the measured impedance.

10. The apparatus according to claim 2, wherein the locking element (350) is shaped to define a ball.

11. The apparatus according to claim 2, wherein the locking element (350) is in electrical contact with the first wire (562), but is not fixed to the first wire (562).

12. The apparatus according to claim 11, wherein the locking element (350) has an outer surface having first and second portions (357, 355), the first portion (357) being non-insulated and in electrical contact with the first wire (562), the second portion (355) being insulated.

13. The apparatus according to claim 12, wherein the locking element (350) is not in electrical contact with any component of the apparatus except via the first wire (562) or the implantable neural stimulator (320).

14. The apparatus according to any one of claims 1-2, further comprising an oral surgical guide (40) generated by using CT scan data of the subject, and comprising:
an arch portion (59) configured to be placed on a dental arch of a subject; and
an extension portion (58) extending from the arch portion (59), and shaped to define a guide hole (6) configured to guide the stimulator (320) through a greater palatine foramen of a palate of an oral cavity of the subject and into the greater palatine canal at an angle that is suitable for entering the greater palatine canal,
wherein the disposition tracker (595) is configured to (a) receive as an input an indication of the angle, and (b) determine the disposition of the implantable neural stimulator (320) based on the indication of the angle.

## Patentansprüche

1. Vorrichtung, umfassend:
einen implantierbaren Nervenstimulator (320) (i) konfiguriert, um durch einen größeren Gaumenkanal zu einem Keilbein-Gaumen-Ganglion (SPG) eines Subjekts vorgerückt zu werden und (ii) umfassend eine Implantat-Impedanz-Erfassungselektrode (560);
eine Hilfs-Impedanz-Erfassungselektrode (580);
einen ersten und einen zweiten Draht (562, 564), welche jeweils elektrisch mit der Implantat-Impedanz-Erfassungselektrode und der Hilfs-Impedanz-Erfassungselektrode (560, 580); und
Impedanz-basierte Navigationsschaltungen (540), umfassend:
einen Spannungsgenerator (660), welcher konfiguriert ist, um einen Strom zwischen der Implantat-Impedanz-Erfassungselektrode und der Hilfs-Impedanz-Erfassungselektrode (560, 580) durch die Drähte (562, 564);
einen Impedanz-Sensor (590), welcher konfiguriert ist, um eine Impedanz zwischen der Implantat-Impedanz-Erfassungselektrode und der Hilfs-Impedanz-Erfassungselektrode (560, 580) basierend auf das Anwenden des Stroms zu messen; und
einen Anordnungsverfolger (595), welcher konfiguriert ist, um, basierend auf eine Änderung der gemessenen Impedanz, eine Anordnung des implantierbaren Nervenstimulators (320) in dem größeren Gaumenkanal zu bestimmen,
wobei die Vorrichtung **dadurch gekennzeichnet ist, dass** die Anordnung des implantierbaren Nervenstimulators (320) eine Form des implantierbaren Nervenstimulators (320) in dem größeren Gaumenkanal umfasst, und wobei der Anordnungsverfolger (595) konfiguriert ist, um die Form des implantierbaren Nervenstimulators (320) in dem größeren Gaumenkanal basierend auf eine Änderung der gemessenen Impedanz zu bestimmen.

2. Vorrichtung nach Anspruch 1, ferner umfassend:
ein Verriegelungselement (350), welches elektrisch den ersten Draht (562) mit der Implantat-Impedanz-Erfassungselektrode (560) koppelt; und
eine Verriegelungselementsteuerung, welche konfiguriert ist, um das Verriegelungselement (350) von dem ersten Draht (562) zu entkoppeln.

3. Vorrichtung nach einem der Ansprüche 1-2, wobei der erste Draht (562) konfiguriert ist, um von der Implantat-Impedanz-Erfassungselektrode (560) entkoppelt zu werden, sobald der Nervenstimulator (320) an den SPG bereitgestellt wird, und wobei der implantierbare Nervenstimulator (320) Stimulationsschaltungen umfasst, welche konfiguriert sind, um die Implantat-Impedanz-Erfassungselektrode (560) so zu treiben, dass diese eine elektrische Stimulation auf den Keilbein-Gaumen-Ganglion (SPG) des Subjekts anwendet, sobald der Nervenstimulator (320) an den SPG bereitgestellt wird, nach dem Entkoppeln des ersten Drahts (562) von der Implantat-Impedanz-Erfassungselektrode (560).

4. Vorrichtung nach einem der Ansprüche 1-2, wobei der implantierbare Nervenstimulator (320) ferner umfasst:
mindestens zwei Stimulationselektroden (12, 14); und
Stimulationsschaltungen, welche konfiguriert sind, um die mindestens zwei Stimulationselektroden (12, 14) zu treiben, um eine elektrische Stimulation auf den Keilbein-Gaumen-Ganglion (SPG) des Subjekts anzuwenden,
wobei der implantierbare Nervenstimulator (320) keine Schaltung zum Treiben der Implantat-Impedanz-Erfassungselektrode (560) umfasst, um eine elektrische Stimulation auf den Keilbein-Gaumen-Ganglion (SPG) des Subjekts anzuwenden.

5. Vorrichtung nach einem der Ansprüche 1-2, ferner umfassend ein Einführinstrument (700), welches konfiguriert ist, um den implantierbaren Nervenstimulator (320) durch den größeren Gaumenkanal distal vorzurücken, wobei die Hilfs-Impedanz-Erfassungselektrode (580) mit dem Einführinstrument (700) gekoppelt ist.

6. Vorrichtung nach Anspruch 1, wobei der Anordnungsverfolger (595) konfiguriert ist, um die Form des implantierbaren Nervenstimulators (320) durch Verfolgen von aufeinanderfolgenden Positionen des implantierbaren Nervenstimulators (320) zu bestimmen.

7. Vorrichtung nach Anspruch 1, wobei:
der implantierbare Nervenstimulator (320) einen proximalen Abschnitt, einen distalen Abschnitt und einen mittleren Abschnitt zwischen dem proximalen und distalen Abschnitt aufweist, wobei der proximale und der distale Abschnitt steifer sind als der mittlere Abschnitt,
die Implantat-Impedanz-Erfassungselektrode (560) auf dem distalen Abschnitt des Nervenstimulators (320) angeordnet ist, und
die Hilfs-Impedanz-Erfassungselektrode (580) auf dem proximalen Abschnitt des Nervenstimulators (320) angeordnet ist.

8. Vorrichtung nach Anspruch 1, wobei die Impedanz-basierte Navigationsschaltung (540) konfiguriert ist, zum:
Erzeugen einer Ausgabe, falls (i) ein distaler Abschnitt des implantierbaren Nervenstimulators (320) nicht in dem größeren Gaumenkanal vorrückt und (ii) ein proximaler Abschnitt des implantierbaren Nervenstimulators (320) in dem größeren Gaumenkanal vorrückt, und wobei
Der Anordnungsverfolger (595) konfiguriert ist, um die Form des implantierbaren Nervenstimulators (320) zu bestimmen, basierend auf der Ausgabe.

9. Vorrichtung nach einem der Ansprüche 1-2, wobei die Anordnung des implantierbaren Nervenstimulators (320) eine Position des implantierbaren Nervenstimulators (320) in dem größeren Gaumenkanal umfasst, und wobei der Anordnungsverfolger (595) konfiguriert ist, um die Position des implantierbaren Nervenstimulators (320) in dem größeren Gaumenkanal basierend auf einer Änderung der gemessenen Impedanz zu bestimmen.

10. Vorrichtung nach Anspruch 2, wobei das Verriegelungselement (350) so geformt ist, dass es eine Kugel definiert.

11. Vorrichtung nach Anspruch 2, wobei das Verriegelungselement (350) sich in elektrischem Kontakt mit dem ersten Draht (562) befindet aber mit dem ersten Draht (562) nicht verbunden ist.

12. Vorrichtung nach Anspruch 11, wobei das Verriegelungselement (350) eine Außenfläche aufweist, welche einen ersten und einen zweiten Abschnitt (357, 355) aufweist, wobei der erste Abschnitt (357) nicht isoliert ist und sich in elektrischem Kontakt mit dem ersten Draht (562) befindet, wobei der zweite Abschnitt (355) isoliert ist.

13. Vorrichtung nach Anspruch 12, wobei das Verriegelungselement (350) sich nicht in elektrischen Kontakt mit irgendeiner Komponente der Vorrichtung, außer durch den ersten Draht (562) oder den implantierbaren Nervenstimulator (320).

14. Vorrichtung nach einem der Ansprüche 1-2, ferner umfassend eine orale chirurgische Führung (40), welche unter Verwendung von CT-Scandaten des Subjekts erzeugt wird, und umfassend:
einen Bogenabschnitt (59), welcher konfiguriert ist, um auf einem Zahnbogen eines Subjekts angeordnet zu werden; und
einen Verlängerungsabschnitt (58), welcher sich vom Bogenabschnitt (59) erstreckt und geformt ist, um ein Führungsloch (6) zu definieren, welches konfiguriert ist, um den Stimulator (320) durch ein größeres Gaumen-Foramen eines Gaumens einer Mundhöhle des Subjekts und in den größeren Gaumenkanal in einem Winkel zu führen, welcher zum Eintreten in den größeren Gaumenkanal geeignet ist,
wobei der Anordnungsverfolger (595) konfiguriert ist, um (a) als Eingabe eine Winkelangabe zu empfangen, und (b) um die Anordnung des implantierbaren Nervenstimulators (320) basierend auf der Winkelangabe zu bestimmen.

## Revendications

1. Appareil comprenant :
un stimulateur neural implantable (320) (i) configuré pour être avancé à travers un conduit grand palatin vers un ganglion sphénopalatin (SPG) d'un sujet et (ii) comprenant une électrode de détection d'impédance d'implant (560) ;
une électrode de détection d'impédance auxiliaire (580) ;
des premier et second fils (562, 564), couplés électriquement respectivement aux électrodes de détection d'impédance d'implant et de détection d'impédance auxiliaire (560, 580) ; et
un circuit de navigation se basant sur l'impédance (540) comprenant :
un générateur de tension (660) configuré pour appliquer un courant entre les électrodes de détection d'impédance d'implant et de détection d'impédance auxiliaire (560, 580) à travers les fils (562, 564) ;
un détecteur d'impédance (590) configuré pour mesurer une impédance entre les électrodes de détection d'impédance d'implant et de détection d'impédance auxiliaire (560, 580) en se basant sur l'application du courant ; et
un pisteur de disposition (595) configuré pour déterminer, en se basant sur un changement dans l'impédance mesurée, une disposition du stimulateur neural implantable (320) dans le conduit grand palatin,
dans lequel l'appareil est **caractérisé en ce que** la disposition du stimulateur neural implantable (320) inclut une forme du stimulateur neural implantable (320) dans le conduit grand palatin, et dans lequel le pisteur de disposition (595) est configuré pour déterminer la forme du stimulateur neural implantable (320) dans le conduit grand palatin en se basant sur un changement dans l'impédance mesurée.

2. Appareil selon la revendication 1, comprenant en outre :
un élément de blocage (350) couplant électriquement le premier fil (562) à l'électrode de détection d'impédance d'implant (560) ; et
un dispositif de commande d'élément de blocage configuré pour désengager l'élément de blocage (350) du premier fil (562).

3. Appareil selon l'une quelconque des revendications 1 et 2, dans lequel le premier fil (562) est configuré pour être découplé de l'électrode de détection d'impédance d'implant (560) une fois que le stimulateur neural (320) a été délivré au SPG, et dans lequel le stimulateur neural implantable (320) comprend un circuit de stimulation configuré pour entraîner l'électrode de détection d'impédance d'implant (560) pour appliquer une stimulation électrique au ganglion sphénopalatin (SPG) du sujet une fois que le stimulateur neural (320) a été délivré au SPG, à la suite du découplage du premier fil (562) de l'électrode de détection d'impédance d'implant (560).

4. Appareil selon l'une quelconque des revendications 1 et 2, dans lequel le stimulateur neural implantable (320) comprend en outre :
au moins deux électrode de stimulation (12, 14) ; et
un circuit de stimulation configuré pour entraîner les au moins deux électrodes de stimulation (12, 14) pour appliquer une stimulation électrique au ganglion sphénopalatin (SPG) du sujet,
dans lequel le stimulateur neural implantable (320) ne comprend pas de circuit pour entraîner l'électrode de détection d'impédance d'implant (560) pour appliquer une stimulation électrique au ganglion sphénopalatin (SPG) du sujet.

5. Appareil selon l'une quelconque des revendications 1 et 2, comprenant en outre un outil de délivrance (700) configuré pour avancer de manière distale le stimulateur neural implantable (320) à travers le conduit grand palatin, dans lequel l'électrode de détection d'impédance auxiliaire (580) est couplée à l'outil de délivrance (700).

6. Appareil selon la revendication 1, dans lequel le pisteur de disposition (595) est configuré pour déterminer la forme du stimulateur neural implantable (320) par le pistage d'emplacements successifs du stimulateur neural implantable (320).

7. Appareil selon la revendication 1, dans lequel :
le stimulateur neural implantable (320) présente une partie proximale, une partie distale et une partie médiane entre les parties proximale et distale, les parties proximale et distale étant plus rigides que la partie médiane,
l'électrode de détection d'impédance d'implant (560) est disposée sur la partie distale du stimulateur neural (320), et
l'électrode de détection d'impédance auxiliaire (580) est disposée sur la partie proximale du stimulateur neural (320).

8. Appareil selon la revendication 1, dans lequel le circuit de navigation se basant sur l'impédance (540) est configuré pour :
générer une sortie si (i) une partie distale du stimulateur neural implantable (320) n'avance pas dans le conduit grand palatin et (ii) une partie proximale du stimulateur neural implantable (320) avance dans le conduit grand palatin, et dans lequel
le pisteur de disposition (595) est configuré pour déterminer la forme du stimulateur neural implantable (320) en se basant sur la sortie.

9. Appareil selon l'une quelconque des revendications 1 et 2, dans lequel la disposition du stimulateur neural implantable (320) inclut un emplacement du stimulateur neural implantable (320) dans le conduit grand palatin, et dans lequel le pisteur de disposition (595) est configuré pour déterminer l'emplacement du stimulateur neural implantable (320) dans le conduit grand palatin en se basant sur un changement dans l'impédance mesurée.

10. Appareil selon la revendication 2, dans lequel l'élément de blocage (350) est façonné pour définir une balle.

11. Appareil selon la revendication 2, dans lequel l'élément de blocage (350) est en contact électrique avec le premier fil (562), mais n'est pas fixé au premier fil (562).

12. Appareil selon la revendication 11, dans lequel l'élément de blocage (350) présente une surface externe présentant des première et seconde parties (357, 355), la première partie (357) n'étant pas isolée et étant en contact électrique avec le premier fil (562), la seconde partie (355) étant isolée.

13. Appareil selon la revendication 12, dans lequel l'élément de blocage (350) n'est pas en contact électrique avec un quelconque composant de l'appareil sauf via le premier fil (562) ou le stimulateur neural implantable (320).

14. Appareil selon l'une quelconque des revendications 1 et 2, comprenant en outre un guide chirurgical oral (40) généré en utilisant des données d'examen tomodensitométrique du sujet, et comprenant :
une partie arcade (59) configurée pour être placée sur une arcade dentaire d'un sujet ; et
une partie en extension (58) s'étendant à partir de la partie arcade (59), et façonnée pour définir un trou de guidage (6) configuré pour guider le stimulateur (320) à travers un foramen grand palatin d'un palais d'une cavité buccale du sujet et dans le conduit grand palatin selon un angle qui est adéquat pour entrer dans le conduit grand palatin,
dans lequel le pisteur de disposition (595) est configuré pour (a) recevoir en tant qu'entrée une indication de l'angle, et (b) déterminer la disposition du stimulateur neural implantable (320) en se basant sur l'indication de l'angle.
